(19) Europäisches Patentamt
European Patent Office
Office européen des brevets

(11) **EP 4 745 282 A1**

(12) **EUROPEAN PATENT APPLICATION**

(43) Date of publication:
20.05.2026 Bulletin 2026/21

(21) Application number: 24213146.4

(22) Date of filing: **15.11.2024**

(51) International Patent Classification (IPC):
**D04H 1/4291** (2012.01)   **D04H 1/4274** (2012.01)
**D04H 3/007** (2012.01)   **D04H 3/16** (2006.01)
**C08F 8/00** (2006.01)   **C08F 210/06** (2006.01)
**C08L 23/12** (2006.01)

(52) Cooperative Patent Classification (CPC):
**D04H 1/4291; C08F 8/00; D04H 1/4274;
D04H 3/007; D04H 3/16**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **The Procter & Gamble Company**
**Cincinnati, OH 45202 (US)**

(72) Inventors:
• **ROSATI, Rodrigo**
**65824 Schwalbach am Taunus (DE)**

• **WEISMAN, Paul Thomas**
**Cincinnati, 45202 (US)**
• **BOND, Eric Bryan**
**Cincinnati, 45202 (US)**
• **CHENG, Calvin Hoi Wung**
**Cincinnati, 45202 (US)**
• **Simonyan, Arsen**
**65824 Schwalbach am Taunus (DE)**

(74) Representative: **Elkington and Fife LLP**
**Prospect House**
**8 Pembroke Road**
**Sevenoaks, Kent TN13 1XR (GB)**

(54)   **NONWOVEN**

(57)   A nonwoven, a method for making such a nonwoven and the use of waste carbon to make such a nonwoven. The nonwoven has a total carbon content (TCC) from which 10% to 100% of the TCC of the nonwoven are derived from waste carbon. The nonwoven meets one or more of the criteria a) to e) by comprising less than

a) 5000 μg/kg of iso-Nonylphenolhexaethoxylate;
b) 500 μg/kg of iso-Nonylphenol;
c) 4.0 ng/kg of Octachlorodibenzodioxin;
d) 500 μg/kg of Di-2-ethylhexyl phthalate; and/ or
e) 25 μg/kg of Napthalene.

Fig. 1

**Description**

FIELD

The present disclosure is generally directed to a nonwoven and a method of making such a nonwoven containing carbon derived from waste carbon and exhibiting low levels of trace chemicals and having acceptable mechanical, visual, tactile, and/or chemical properties.

BACKGROUND

Nonwoven webs are used in many industries, including the medical, hygiene, and cleaning industries. Absorbent articles comprising nonwoven webs are used in the hygiene industry to contain and absorb bodily exudates (i.e., urine, bowel movements, and menses) in infants, toddlers, children, and adults. Absorbent articles may include, but not be limited to, diapers, pants, adult incontinence products, feminine care products, and absorbent pads. Various components of these absorbent articles comprise nonwoven webs. Some example components that comprise nonwoven webs are outer cover nonwoven materials, topsheets, waistbands, leg cuffs, waist cuffs, ears, belts, and acquisition materials, for example.

There is an ever pressing need to reduce the environmental impact of consumer products. Nonwovens are typically made with virgin petrol-based carbon materials manufactured into chemical structures commonly called polymers, such as polyethylene (PE), polypropylene (PP) and/or polyethylene terephthalate (PET). However, fossil fuels are a finite resource and global demand for petroleum continues to increase due to growing populations and increasing standards of living worldwide. In addition, consumer demand for products and packaging made at least partially from renewable and/or recycled resources has increased significantly over the past decade, and has become a driver of innovation for new and improved consumer products and packaging materials. In the context of nonwovens, one way to address this consumer demand is to replace at least a proportion of virgin petrol-based carbon material with materials derived from waste carbon, in particular recycled carbon material. Such a replacement of virgin materials by materials, which have served a first life, is also desirable from a sustainability point of view.

Adding waste and/or recycled materials into the nonwoven manufacturing process, however, often negatively impacts the quality of the resulting nonwoven or even renders the manufacturing process impossible or prone to outages. Negative impacts on quality can manifest in a number of properties of the nonwoven, such as diminished mechanical properties, undesirable visual and tactile properties of the nonwoven. Additionally, nonwovens, in particular when used in the context of absorbent articles, are often in direct or indirect contact with the skin of the consumer and/ or caretaker. Hence, nonwovens comprising materials derived from waste carbon are required to be free of or contain minimal amounts of contaminants and/or trace chemicals.

Net, there is a need for nonwovens comprising materials derived from waste carbon, thus nonwovens with improved sustainability, exhibiting low contaminant and/or trace chemicals levels and maintaining the required mechanical properties.

SUMMARY

The present disclosure provides a nonwoven having a total carbon content (TCC). From 10% to 100% of the TCC of the nonwoven are derived from waste carbon. The nonwoven meets one or more of the criteria a) to e) by comprising less than

a) 5000 μg/kg, preferably less than 100 μg/kg of iso-Nonylphenolhexaethoxylate;
b) 500 μg/kg, preferably less than 100 μg/kg of iso-Nonylphenol;
c) 4.0 ng/kg, preferably less than 0.4 ng/kg of Octachlorodibenzodioxin;
d) 500 μg/kg, preferably less than 100 μg/kg of Di-2-ethylhexyl phthalate; and/ or
e) 25 μg/kg, preferably less than 5 μg/kg of Napthalene;

according to the Trace Chemicals Test Method described herein.

Further, the present disclosure provides the use of waste carbon to make a nonwoven having a total carbon content (TCC). From 10% to 100% of the TCC of the nonwoven are waste carbon. The nonwoven meets one or more of the criteria a) to e) by comprising less than

a) 5000 μg/kg, preferably less than 100 μg/kg of iso-Nonylphenolhexaethoxylate;
b) 500 μg/kg, preferably less than 100 μg/kg of iso-Nonylphenol;
c) 4.0 ng/kg, preferably less than 0.4 ng/kg of Octachlorodibenzodioxin;

d) 500 μg/kg, preferably less than 100 μg/kg of Di-2-ethylhexyl phthalate; and/ or

e) 25 μg/kg, preferably less than 5 μg/kg of Napthalene;

according to the Trace Chemicals Test Method described herein.

Finally, the present disclosure provides a method for making a nonwoven having a total carbon content (TCC). In one step, waste carbon is provided. In the produced nonwoven from 10% to 100% of the TCC derive from the provided waste carbon. The produced nonwoven meets one or more of the criteria a) to e) by comprising less than

a) 5000 μg/kg, preferably less than 100 μg/kg of iso-Nonylphenolhexaethoxylate;

b) 500 μg/kg, preferably less than 100 μg/kg of iso-Nonylphenol;

c) 4.0 ng/kg, preferably less than 0.4 ng/kg of Octachlorodibenzodioxin;

d) 500 μg/kg, preferably less than 100 μg/kg of Di-2-ethylhexyl phthalate; and/ or

e) 25 μg/kg, preferably less than 5 μg/kg of Napthalene;

according to the Trace Chemicals Test Method described herein.

BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:

Fig. 1 is a plan view of an example absorbent article in the form of a taped diaper, garment-facing surface facing the viewer, in a flat laid-out state;

Fig. 2 is a plan view of the example absorbent article of Fig. 1, wearer-facing surface facing the viewer, in a flat laid-out state;

Fig. 3 is a front perspective view of the absorbent article of Figs. 1 and 2 in a fastened position;

Fig. 4 is a front perspective view of an absorbent article in the form of a pant;

Fig. 5 is a rear perspective view of the absorbent article of Fig. 4;

Fig. 6 is a plan view of the absorbent article of Fig. 4, laid flat, with a garment-facing surface facing the viewer;

Fig. 7 is a cross-sectional view of the absorbent article taken about line 7-7 of Fig. 6;

Fig. 8 is a cross-sectional view of the absorbent article taken about line 8-8 of Fig. 6;

Fig. 9 is a plan view of an example absorbent core or an absorbent article;

Fig. 10 is a cross-sectional view, taken about line 10-10, of the absorbent core of Fig. 9;

Fig. 11 is a cross-sectional view, taken about line 11-11, of the absorbent core of Fig. 9;

Fig. 12 is a plan view of an example absorbent article of the present disclosure that is a sanitary napkin; and

Fig. 13 is a plan view of an example absorbent article in the form of a taped diaper, garment-facing surface facing the viewer, in a flat laid-out state.

DETAILED DESCRIPTION

Various non-limiting forms of the present disclosure will now be described to provide an overall understanding of the principles of the structure, function, manufacture, and use of the nonwoven disclosed herein. One or more examples of these non-limiting forms are illustrated in the accompanying drawings. Those of ordinary skill in the art will understand that the nonwoven described herein and illustrated in the accompanying drawings are non-limiting example forms and that the scope of the various non-limiting forms of the present disclosure are defined solely by the claims. The features illustrated or described in connection with one non-limiting form may be combined with the features of other non-limiting forms. Such modifications and variations are intended to be included within the scope of the present disclosure.

Definition of Terms

The term "absorbent article" may include disposable articles such as sanitary napkins, panty liners, tampons, interlabial devices, wound dressings, pants, taped diapers, adult incontinence articles, wipes, and the like. At least some of such absorbent articles are intended for the absorption of body liquids, such as menses or blood, vaginal discharges, urine, and feces. Wipes may be used to absorb body liquids, or may be used for other purposes, such as for cleaning surfaces. The nonwoven materials described herein may comprise at least part of other articles such as scouring pads, wet or dry-mop pads (such as SWIFFER® pads), paper towels, toilet tissue, and the like.

The term "disposable" may be used herein to describe absorbent articles and other products which are not intended to be laundered or otherwise restored or reused as an absorbent article or product (i.e., they are intended to be discarded after use and, preferably, to be recycled, composted or otherwise disposed of in an environmentally compatible manner).

As used herein, "virgin material" refers to material produced directly from the petrochemical or plant-based feedstock, such as natural gas, crude oil, or sugar cane, and which has never been used or processed before the initial structure manufacturing process.

As used herein, "recycled carbon" and "recycled material" refer to carbon and/ or material produced directly from materials other than virgin petrochemical and/ or virgin plant-based feedstock, such as material previously formed into films, containers, fibers, nonwovens or other goods.

The term "machine direction" or "MD" may be used herein to refer to the direction of material flow through a manufacturing process. In addition, relative placement and movement of material can be described as flowing in the machine direction through a process from upstream in the process to downstream in the process. The term "cross direction" or "CD" may be used herein to refer to a direction that is generally perpendicular to the machine direction. MD and CD can also be used in reference to orientation of properties in a nonwoven. MD strength, for example, reference to the strength properties on the nonwoven in the machine direction, the direction the nonwoven was traveling during production.

As used herein, "high density polyethylene" (HDPE) means a type of polyethylene defined by a density equal to or greater than $0.941 g/cm^3$.

As used herein, "low density polyethylene" (LDPE) means a type of polyethylene defined by a density equal to or less than $0.925 g/cm^3$.

As used herein, "medium density polyethylene" (MDPE) means a type of polyethylene defined by a density range of $0.926-0.940 g/cm^3$.

As used herein, "linear low density polyethylene" (LLDPE) means a type of Low Density Polyethylene characterized by substantially linear polyethylene, with significant numbers of short branches, commonly made by copolymerization of ethylene with longer-chain olefins. Linear low-density polyethylene differs structurally from conventional low-density polyethylene (LDPE) because of the absence of long chain branching. The linearity of LLDPE results from the different manufacturing processes of LLDPE and LDPE. In general, LLDPE is produced at lower temperatures and pressures by copolymerization of ethylene and such higher alpha-olefins as butene, hexene, or octene. The copolymerization process produces a LLDPE polymer that has a narrower molecular weight distribution than conventional LDPE and in combination with the linear structure, significantly different rheological properties.

As used herein, "waste carbon" means carbon contained in materials that have been processed into a useable form and served their first life. In that respect, waste carbon can be derived from waste fossil-derived materials (e.g., used plastics), waste plant-derived materials (e.g., used cooking oils), and carbon dioxide in the atmosphere (e.g. emitted from incineration of waste). Hence, waste carbon containing materials can be produced with different methods. For example, they can be produced from used plastics via physical or chemical recycling, from used cooking oil via chemical transformations such as hydrodeoxygenation and isomerization, from carbon dioxide in the atmosphere via carbon capture and utilization approaches.

As used herein, "virgin-like" refers to the material used in the present invention and is an outcome of the purification process. "virgin-like" can be used in reference to the purified pellets or nonwovens made from these pellets. The processing of "virgin-like" pellets does not alter the chemical composition. "virgin-like" resin may also contain standard stabilizer packages found in "virgin-material" at similar levels. Just to eliminate any uncertainty, virgin and "virgin-like have different definitions as described herein.

Nonwoven

A nonwoven substantially free of contaminants was obtained from waste carbon. The level of five contaminants/ trace chemicals may be determined. The contaminants/ trace chemicals iso-Nonylphenolhexaethoxylate, iso-Nonylphenol, Octachlorodibenzodioxin, Di-2-ethylhexyl phthalate (DEHP) and Napthtalene are representative substances of interest, which should be avoided in the context of nonwovens, and belong to typical families of compounds found in waste material and material obtained from recycling waste.

A nonwoven is provided having a total carbon content (TCC). For the total carbon content (TCC), the carbon contained in nonwovens is determined as moles per 100g nonwoven material according to ASTM D5291-16 (Method C) with an accuracy of 0.01 moles of carbon per 100g of nonwoven material. From 10% to 100% of the TCC of the nonwoven are derived from waste carbon. The nonwoven may comprise from 0.72 to 7.20 moles of carbon per 100g of nonwoven, or from 5.76 to 7.20 moles of carbon per 100g of nonwoven, or from 6.84 to 7.20 moles of carbon per 100g of nonwoven, or from 6.98 to 7.20 moles of carbon per 100g of nonwoven, or from 7.13 to 7.20 moles of carbon per 100g of nonwoven. From 80% to 100%, preferably from 90% to 100%, more preferably from 95% to 100%, even more preferably from 97% to 100% or even from 99% to 100% of the TCC of the nonwoven may be derived from waste carbon. The waste carbon may preferably be post-consumer recycled carbon.

The nonwoven may comprise polyolefins, such as polypropylene or polyethylene, and/ or other polymeric material, such as polyesters, in particular PET (polyethylene terephthalate). The nonwoven may have a polyolefin content of at least 90% by weight of the nonwoven. The nonwoven may comprise one or more of low density polyethylene, medium density polyethylene high density polyethylene, linear low density polyethylene, a propylene homopolymer, an ethylene copolymer, or a propylene copolymer or one or more blends thereof. For example, the nonwoven may have a PP content of at least 90% by weight of the nonwoven.

The nonwoven may comprise from 20 weight-% to 100 weight-%, preferably from 40 weight-% to 99.8 weight-%, more preferably from 60 weight-% to 99.5 weight-%, even more preferably from 80 weight-% to 99.3 weight-%, even more preferably from 90 weight-% to 99.0 weight-%, or even from 95 weight-% to 98.5 weight-% polyolefin by weight of the nonwoven. The nonwoven may comprise from 20 weight-% to 100 weight-%, preferably from 40 weight-% to 99.8 weight-%, more preferably from 60 weight-% to 99.5 weight-%, even more preferably from 80 weight-% to 99.3 weight-%, even more preferably from 90 weight-% to 99.0 weight-%, or even from 95 weight-% to 98.5 weight-% recycled polyolefin by weight of the nonwoven, preferably post-consumer recycled polyolefin. From 80 weight-% to 100 weight-%, preferably from 90 weight-% to 100 weight-%, more preferably from 95 weight-% to 100 weight-%, even more preferably from 97 weight-% to 100 weight-% or even from 98 weight-% to 100 weight-% by weight of the total polyolefin may be recycled polyolefin. From 80 weight-% to 100 weight-%, preferably from 90 weight-% to 100 weight-%, more preferably from 95 weight-% to 100 weight-%, even more preferably from 97 weight-% to 100 weight-% or even from 98 weight-% to 100 weight-% by weight of the total recycled polyolefin may be post-consumer recycled (PCR) polyolefin. From 80 weight-% to 100 weight-%, preferably from 90 weight-% to 100 weight-%, more preferably from 95 weight-% to 100 weight-%, even more preferably from 97 weight-% to 100 weight-% or even from 98 weight-% to 100 weight-% by weight of the total polyolefin may be post-consumer recycled (PCR) polyolefin.

The nonwoven comprises less than 5000 $\mu$g/kg, preferably less than 100 $\mu$g/kg, more preferably less than 50 $\mu$g/kg, even more preferably less than 20 $\mu$g/kg of iso-Nonylphenolhexaethoxylate. The nonwoven may comprise from 0 $\mu$g/kg to 5000 $\mu$g/kg, preferably from 0 $\mu$g/kg to 100 $\mu$g/kg, more preferably from 0 $\mu$g/kg to 50 $\mu$g/kg, even more preferably from 0 $\mu$g/kg to 20 $\mu$g/kg of iso-Nonylphenolhexaethoxylate. In addition or alternatively, the nonwoven comprises less than 500 $\mu$g/kg, preferably less than 100 $\mu$g/kg, more preferably less than 50 $\mu$g/kg, even more preferably less than 20 $\mu$g/kg of iso-Nonylphenol. The nonwoven may comprise from 0 $\mu$g/kg to 500 $\mu$g/kg, preferably from 0 $\mu$g/kg to 100 $\mu$g/kg, more preferably from 0 $\mu$g/kg to 50 $\mu$g/kg, even more preferably from 0 $\mu$g/kg to 20 $\mu$g/kg of iso-Nonylphenol. Nonylphenol derivates are widely used as antioxidants, lubricating oil additives, detergents, emulsifiers, and/or solubilizers. In addition or alternatively, the nonwoven comprises less than 4.0 ng/kg, preferably less than 0.4 ng/kg, more preferably less than 0.2 ng/kg even more preferably less than 0.1 ng/kg of Octachlorodibenzodioxin. The nonwoven may comprise from 0.0 ng/kg to 4.0 ng/kg, preferably from 0.0 ng/kg to 0.4 ng/kg, more preferably from 0.0 ng/kg to 0.2 ng/kg even more preferably from 0.0 ng/kg to 0.1 ng/kg of Octachlorodibenzodioxin. Octachlorodibenzodioxin can originate from water treatment, bleaching processes and/ or as by-product of the manufacture of organochlorides. In addition or alternatively, the nonwoven comprises less than 500 $\mu$g/kg, preferably less than 100 $\mu$g/kg, more preferably less than 50 $\mu$g/kg, even more preferably less than 20 $\mu$g/kg of Di-2-ethylhexyl phthalate (DEHP). The nonwoven may comprise from 0 $\mu$g/kg to 500 $\mu$g/kg, preferably from 0 $\mu$g/kg to 100 $\mu$g/kg, more preferably from 0 $\mu$g/kg to 50 $\mu$g/kg, even more preferably from 0 $\mu$g/kg to 20 $\mu$g/kg of DEHP. DEHP is employed as plasticizer. In addition or alternatively, the nonwoven comprises less than 25 $\mu$g/kg, preferably less than 5 $\mu$g/kg, more preferably less than 3 $\mu$g/kg, even more preferably less than 2 $\mu$g/kg of Napthalene. The nonwoven may comprise from 0 $\mu$g/kg to 25 $\mu$g/kg, preferably from 0 $\mu$g/kg to 5 $\mu$g/kg, more preferably from 0 $\mu$g/kg to 3 $\mu$g/kg, even more preferably from 0 $\mu$g/kg to 2 $\mu$g/kg of Naphtalene. Naphtalene has many applications as plasticizers, as dispersants, as tanning agents etc.

The nonwoven comprises at least one trace chemical, preferably at least two trace chemicals, more preferably at least three trace chemicals, even more preferably at least four trace chemicals or even all five trace chemicals in amounts below the levels specified and may not comprise detectable amounts of the trace chemicals mentioned. The trace chemical levels are determined according to the Trace Chemicals Test Method described below.

The nonwoven may exhibit a machine direction (MD) to cross direction (CD) strength ratio of from 1.0 to 5.0, preferably from 1.2 to 3.5, more preferably from 1.5 to 2.5, even more preferably from 1.8 to 2.2 according to the Tensile Strength Test method described herein.

The nonwoven may exhibit an opacity of from 5% to 70%, preferably from 10% to 60%, more preferably from 15 to 55% according to the Color and Opacity Measurement.

The nonwoven may be an essentially white nonwoven. The nonwoven may exhibit a L* value of at least 85, preferably at least 88, more preferably at least 90, even more preferably at least 92, most preferably at least 94 or even at least 96 according to the Color and Opacity Measurement. The nonwoven may exhibit an a* value of from -20 to 20, preferably from -15 to 15, more preferably from -10 to 10, even more preferably from -5 to 5, most preferably from -3 to 3 or even around 0 according to the Color and Opacity Measurement. The nonwoven may exhibit a b* value of from -20 to 30, preferably from -15 to 20, more preferably from -10 to 10, even more preferably from -5 to 5, most preferably from -3 to 3 or even around 0 according to the Color and Opacity Measurement. Thus, the nonwoven may meet opacity and/ or color requirements despite being based on waste carbon. Without being bound to theory, it is believed that due to vis-breaking, colored compounds, such as organic colorants, may be oxidized and thus the coloring reduced.

An absorbent article may comprise the nonwoven described herein.

Recycled carbon and resin

Plastics recycling has emerged as one solution to mitigate the issues associated with the widespread usage of plastics. Recovering and re-using plastics diverts waste from landfills and reduces the demand for virgin plastics made from fossil-based resources, which consequently reduces greenhouse gas emissions. In developed regions, such as the United States and the European Union, rates of plastics recycling are increasing due to greater awareness by consumers, businesses, and industrial manufacturing operations. The majority of recycled materials, including plastics, are mixed into a single stream which is collected and processed by a material recovery facility (MRF). At the MRF, materials are sorted, washed, and packaged for resale. Plastics can be sorted into individual materials, such as high-density polyethylene (HDPE) or poly(ethylene terephthalate) (PET), or mixed streams of other common plastics, such as polypropylene (PP), low-density polyethylene (LDPE), poly(vinyl chloride) (PVC), polystyrene (PS), polycarbonate (PC), and polyamides (PA). The single or mixed streams can then be further sorted, washed, and reprocessed into a pellet that is suitable for re-use in plastics processing, for example blow and injection molding.

Recycling of polymeric carbon materials for making new products or materials is widely known. Generally, it is distinguished between preconsumer/ post-industrial recycled material (also referred to as PIR) and post-consumer recycled material (also referred to as PCR), e.g. as described in ISO norm 14021:2016(E). PCR material is waste created by consumers after a product has reached the end of its use. This material is diverted from the landfill or recuperated in recollection systems and utilized in the production of other commodities. In contrast to PCR materials, PIR relates to reintroduction of waste generated in a manufacturing process back into the original manufacturing process.

Though recycled plastics are sorted into predominately uniform streams and are washed with aqueous and/or caustic solutions, the final reprocessed pellet often remains highly contaminated with unwanted waste impurities, such as spoiled food residue and residual perfume components. In addition, recycled plastic pellets, except for those from recycled beverage containers, are darkly colored due to the mixture of dyes and pigments commonly used to colorize plastic articles. In addition to being contaminated with impurities and mixed colorants, many recycled resin products are often heterogeneous in chemical composition and may contain a significant amount of polymeric contamination, such as polyethylene (PE) contamination in recycled PP and vice versa.

Mechanical recycling, also known as secondary recycling, is the process of converting recycled plastic waste into a re-usable form for subsequent manufacturing. A more detailed review of mechanical recycling and other plastics recovery processes are described in S.M. Al-Salem, P. Lettieri, J. Baeyens, "Recycling and recovery routes of plastic solid waste (PSW): A review", Waste Management, Volume 29, Issue 10, October 2009, Pages 2625-2643, ISSN 0956-053X. While advances in mechanical recycling technology have improved the quality of recycled polymers to some degree, there are fundamental limitations of mechanical decontamination approaches, such as the physical entrapment of pigments within a polymer matrix. Thus, even with the improvements in mechanical recycling technology, the dark color and high levels of chemical contamination in currently available recycled plastic waste prevents broader usage of recycled resins by the plastics industry.

The process of obtaining PCR polymeric material typically includes such mechanical recycling comprising the steps of collecting and sorting used products made with a target polymer such as PE, PP and/ or PET, washing the used products, melting the used products into a raw material resin containing the target polymer, and reusing the resin to produce new products. Such resins often contain high levels of contaminants, *inter alia* due to the exposure to environment in the waste stream, which are introduced to the nonwoven produced from the resins. Further, the derived resins do not exhibit target properties, such as melt flow properties, required to be employed in the spinning process yielding the nonwoven.

In one embodiment, the TCC of the nonwoven derived from waste carbon may be derived from recycled carbon. The waste carbon may be derived from recycled PCR material. PCR material may be prone to acquire significant amounts of contamination that may lead to reduced quality when recycled. Thus, it is particular challenging to obtain a resin exhibiting the target properties for spinning and to produce a nonwoven comprising low contaminant levels and meeting the required mechanical, visual and tactile properties for applications e.g. in absorbent articles.

The nonwoven may be made from a resin obtained via solvent recycling of waste carbon. Solvent recycling of waste carbon material is described in US 10,450,436B2 and US 10,442,912B2. A method for solvent recycling of a reclaimed polypropylene or polyethylene may be employed. The method for purifying reclaimed polypropylene or polyethylene may comprise the steps of:

a. Obtaining the reclaimed polypropylene or polyethylene wherein said reclaimed polypropylene or polyethylene is selected from the group consisting of post-consumer use polymers, post-industrial use polymers, and combinations thereof;

b. Contacting the reclaimed polypropylene or polyethylene at a temperature from about 80°C to about 220°C and at a pressure from about 150 psig (1.03 MPa) to about 15,000 psig (103.42 MPa) with a first fluid solvent having a standard boiling point less than about 70°C, to produce an extracted reclaimed polypropylene or polyethylene;

c. Dissolving the extracted reclaimed polypropylene or polyethylene in a solvent selected from the group consisting of the first fluid solvent, a second fluid solvent, and mixtures thereof, at a temperature from about 90°C to about 220°C and a pressure from about 350 psig (2.41 MPa) to about 20,000 psig (137.90 MPa) to produce a first solution comprising polypropylene or polyethylene and suspended contaminants/ trace chemicals;

d. Settling said first solution comprising polyethylene and suspended contaminants/ trace chemicals at a temperature from about 90°C to about 220°C and at a pressure from about 350 psig (2.41 MPa) to about 20,000 psig (137.90 MPa) to produce a second solution comprising polypropylene or polyethylene and remaining contaminants/ trace chemicals;

e. Purifying said second solution at a temperature from about 90°C to about 220°C and at a pressure from about 350 psig (2.41 MPa) to about 20,000 psig (137.90 MPa) by contacting said second solution with solid media to produce a third solution comprising purer polypropylene or polyethylene; and

f. Separating said purer polypropylene or polyethylene from said third solution.

The second fluid solvent may have the same chemical composition or a different chemical composition as the first fluid solvent.

Such solvent recycling leads to recycled material having lower contaminant levels than via mechanical recycling, in particular for PCR materials. However, resins obtained from the solvent recycling process typically do not meet the target properties, such as melt flow properties, for spinning of a nonwoven.

The nonwoven may be made from a resin comprising less than 5000 μg/kg, preferably less than 100 μg/kg, more preferably less than 50 μg/kg, even more preferably less than 20 μg/kg of iso-Nonylphenolhexaethoxylate. The resin may comprise from 0 μg/kg to 5000 μg/kg, preferably from 0 μg/kg to 100 μg/kg, more preferably from 0 μg/kg to 50 μg/kg, even more preferably from 0 μg/kg to 20 μg/kg of iso-Nonylphenolhexaethoxylate. In addition or alternatively, the resin may comprise less than 500 μg/kg, preferably less than 100 μg/kg, more preferably less than 50 μg/kg, even more preferably less than 20 μg/kg of iso-Nonylphenol. The resin may comprise from 0 μg/kg to 500 μg/kg, preferably from 0 μg/kg to 100 μg/kg, more preferably from 0 μg/kg to 50 μg/kg, even more preferably from 0 μg/kg to 20 μg/kg of iso-Nonylphenol. In addition or alternatively, the resin may comprise less than 4.0 ng/kg, preferably less than 0.4 ng/kg, more preferably less than 0.2 ng/kg even more preferably less than 0.1 ng/kg of Octachlorodibenzodioxin. The resin may comprise from 0.0 ng/kg to 4.0 ng/kg, preferably from 0.0 ng/kg to 0.4 ng/kg, more preferably from 0.0 ng/kg to 0.2 ng/kg even more preferably from 0.0 ng/kg to 0.1 ng/kg of Octachlorodibenzodioxin. In addition or alternatively, the resin may comprise less than 500 μg/kg, preferably less than 100 μg/kg, more preferably less than 50 μg/kg, even more preferably less than 20 μg/kg of Di-2-ethylhexyl phthalate (DEHP). The resin may comprise from 0 μg/kg to 500 μg/kg, preferably from 0 μg/kg to 100 μg/kg, more preferably from 0 μg/kg to 50 μg/kg, even more preferably from 0 μg/kg to 20 μg/kg of DEHP. In addition or alternatively, the resin may comprise less than 25 μg/kg, preferably less than 5 μg/kg, more preferably less than 3 μg/kg, even more preferably less than 2 μg/kg of Napthalene. The resin may comprise from 0 μg/kg to 25 μg/kg, preferably from 0 μg/kg to 5 μg/kg, more preferably from 0 μg/kg to 3 μg/kg, even more preferably from 0 μg/kg to 2 μg/kg of Naphtalene. The resin may comprise at least one contaminant/ trace chemicals, preferably at least two contaminants/ trace chemicals, more preferably at least three contaminants/ trace chemicals, even more preferably at least four contaminants/ trace chemicals or even all five contaminants/ trace chemicals in amounts below the levels specified. The contaminant/ trace chemicals levels are determined according to the Trace Chemicals Test Method described herein. The resin may comprise the low level of contaminants/ trace chemicals specified above prior and/ or after being treated with a vis-breaking agent.

The nonwoven may be made from a virgin-like resin treated with a vis-breaking agent. The vis breaking agent may be an oxidizing vis-breaking agent, in particular a peroxide-group containing compound. It has been found that treating a resin obtained from recycling with a vis-breaking agent may yield resins meeting the target properties, such as melt flow properties, for spinning of nonwoven. Surprisingly, despite the chemical reaction and potentially contaminated vis-breaking agents, nonwovens with the described low contaminant/ trace chemical levels were obtained from resins treated with vis-breaking agents. It is assumed that in particular oxidizing vis-breaking agents may lower contaminant levels, as for example dioxanes and phenols may be converted to less harmful substances via oxidation. Typical peroxide containing vis-breaking agent is Milliken r075 or the Polyvel CR-series. The concentration level of the peroxide in the additive is adjusted to provide the needed viscosity modification. There are also non-peroxide viscosity modification materials, such as BASF Irgatec CR76. The viscosity modification can either be done in-line with the nonwoven forming process or done prior to final processing (commonly referred to as precompounding). The viscosity modification is done in the presence of heat. For clarity purposes, external agents may not be required for viscosity modification.

The nonwoven in the present invention may be made from a virgin-like resin having a melt flow rate (MFR) from 10 g/10 min to 100 g/10 min, preferably from 20 g/10 min to 60 g/10min, most preferred from 25 g/10 min to 50 g/10 min according to the Melt Flow Rate Test Method described herein. By such MFR it is ensured that the spinning process for the nonwoven can be readily performed.

The nonwoven may be made from a resin having an Apparent Shear Viscosity from 15 Pa s to 150 Pa s, preferably from 20 Pa s to 120 Pa s, most preferred 30 Pa s to 100 Pa s when measured at 230°C at 500/s shear rate using a length to diameter die of 30:1, according to the Apparent Shear Viscosity Test Method described herein. By such Apparent Shear Viscosity it is ensured that the spinning process for the nonwoven can be readily performed.

Use of waste carbon to make a nonwoven

The use of waste carbon to make a nonwoven as described herein is provided. Waste carbon may be used to make a nonwoven having a total carbon content (TCC); wherein from 10% to 100% of the TCC of the nonwoven are derived from the used waste carbon;

and wherein the nonwoven meets one or more of the criteria a) to e) by comprising less than

a) 5000 $\mu$g/kg, preferably less than 100 $\mu$g/kg of iso-Nonylphenolhexaethoxylate;
b) 500 $\mu$g/kg, preferably less than 100 $\mu$g/kg of iso-Nonylphenol;
c) 4.0 ng/kg, preferably less than 0.4 ng/kg of Octachlorodibenzodioxin;
d) 500 $\mu$g/kg, preferably less than 100 $\mu$g/kg of Di-2-ethylhexyl phthalate; and/ or
e) 25 $\mu$g/kg, preferably less than 5 $\mu$g/kg of Napthalene;

according to the Trace Chemicals Test Method described herein.

In particular, the waste carbon may be used to obtain a resin, as described herein, to make the nonwoven.

Method for making a nonwoven

A method for making a nonwoven as described herein is provided. In a first step waste carbon as described herein is provided. The made nonwoven has total carbon content (TCC) and from 10% to 100% of the TCC of the made nonwoven derive from the provided waste carbon.

The method may further comprise the step of solvent recycling the waste carbon to obtain a resin to make the nonwoven. The solvent recycling purifying reclaimed polypropylene or polyethylene may comprise the steps of:

a. Obtaining the reclaimed polypropylene or polyethylene wherein said reclaimed polypropylene or polyethylene is selected from the group consisting of post-consumer use polymers, post-industrial use polymers, and combinations thereof;
b. Contacting the reclaimed polypropylene or polyethylene at a temperature from about 80°C to about 220°C and at a pressure from about 150 psig (1.03 MPa) to about 15,000 psig (103.42 MPa) with a first fluid solvent having a standard boiling point less than about 70°C, to produce an extracted reclaimed polypropylene or polyethylene;
c. Dissolving the extracted reclaimed polypropylene or polyethylene in a solvent selected from the group consisting of the first fluid solvent, a second fluid solvent, and mixtures thereof, at a temperature from about 90°C to about 220°C and a pressure from about 350 psig (2.41 MPa) to about 20,000 psig (137.90 MPa) to produce a first solution comprising polypropylene or polyethylene and suspended contaminants/ trace chemicals;
d. Settling said first solution comprising polyethylene and suspended contaminants/ trace chemicals at a temperature from about 90°C to about 220°C and at a pressure from about 350 psig (2.41 MPa) to about 20,000 psig (137.90 MPa) to produce a second solution comprising polypropylene or polyethylene and remaining contaminants/ trace chemicals;
e. Purifying said second solution at a temperature from about 90°C to about 220°C and at a pressure from about 350 psig (2.41 MPa) to about 20,000 psig (137.90 MPa) by contacting said second solution with solid media to produce a third solution comprising purer polypropylene or polyethylene; and

f. Separating said purer polypropylene or polyethylene from said third solution.

The second fluid solvent may have the same chemical composition or a different chemical composition as the first fluid solvent.

The nonwoven may be made from a resin comprising less than 5000 μg/kg, preferably less than 100 μg/kg, more preferably less than 50 μg/kg, even more preferably less than 20 μg/kg of iso-Nonylphenolhexaethoxylate. The resin may comprise from 0 μg/kg to 5000 μg/kg, preferably from 0 μg/kg to 100 μg/kg, more preferably from 0 μg/kg to 50 μg/kg, even more preferably from 0 μg/kg to 20 μg/kg of iso-Nonylphenolhexaethoxylate. In addition or alternatively, the resin may comprise less than 500 μg/kg, preferably less than 100 μg/kg, more preferably less than 50 μg/kg, even more preferably less than 20 μg/kg of iso-Nonylphenol. The resin may comprise from 0 μg/kg to 500 μg/kg, preferably from 0 μg/kg to 100 μg/kg, more preferably from 0 μg/kg to 50 μg/kg, even more preferably from 0 μg/kg to 20 μg/kg of iso-Nonylphenol. In addition or alternatively, the resin may comprise less than 4.0 ng/kg, preferably less than 0.4 ng/kg, more preferably less than 0.2 ng/kg even more preferably less than 0.1 ng/kg of Octachlorodibenzodioxin. The resin may comprise from 0.0 ng/kg to 4.0 ng/kg, preferably from 0.0 ng/kg to 0.4 ng/kg, more preferably from 0.0 ng/kg to 0.2 ng/kg even more preferably from 0.0 ng/kg to 0.1 ng/kg of Octachlorodibenzodioxin. In addition or alternatively, the resin may comprise less than 500 μg/kg, preferably less than 100 μg/kg, more preferably less than 50 μg/kg, even more preferably less than 20 μg/kg of Di-2-ethylhexyl phthalate (DEHP). The resin may comprise from 0 μg/kg to 500 μg/kg, preferably from 0 μg/kg to 100 μg/kg, more preferably from 0 μg/kg to 50 μg/kg, even more preferably from 0 μg/kg to 20 μg/kg of DEHP. In addition or alternatively, the resin may comprise less than 25 μg/kg, preferably less than 5 μg/kg, more preferably less than 3 μg/kg, even more preferably less than 2 μg/kg of Napthalene. The resin may comprise from 0 μg/kg to 25 μg/kg, preferably from 0 μg/kg to 5 μg/kg, more preferably from 0 μg/kg to 3 μg/kg, even more preferably from 0 μg/kg to 2 μg/kg of Naphtalene. The resin may comprise at least one contaminant/ trace chemical, preferably at least two contaminants/ trace chemicals, more preferably at least three contaminants/ trace chemicals, even more preferably at least four contaminants/ trace chemicals or even all five contaminants/ trace chemicals in amounts below the levels specified. The contaminant/ trace chemical levels are determined according to the Trace Chemicals Test Method described herein.

The method may further comprise the step of treating a or the resin obtained from waste carbon with a vis-breaking agent. The vis breaking agent may be an oxidizing vis-breaking agent, in particular a peroxide-group containing compound. The resin may comprise the low level of contaminants specified above prior and/ or after the step of being treated with a vis-breaking agent.

General Description of an Absorbent Article

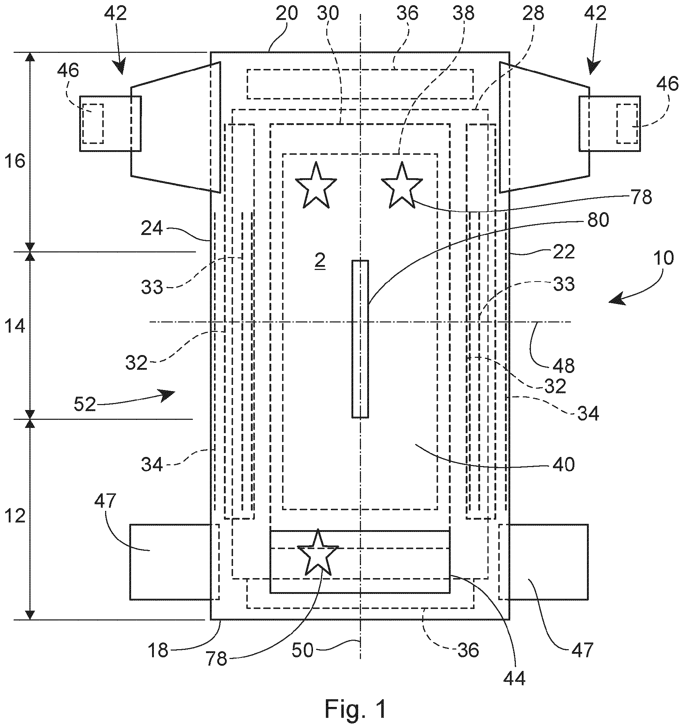

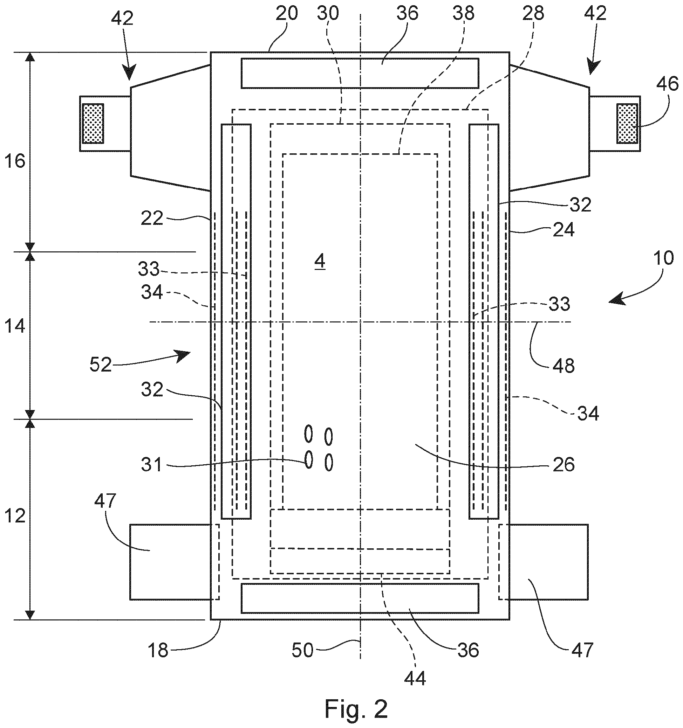

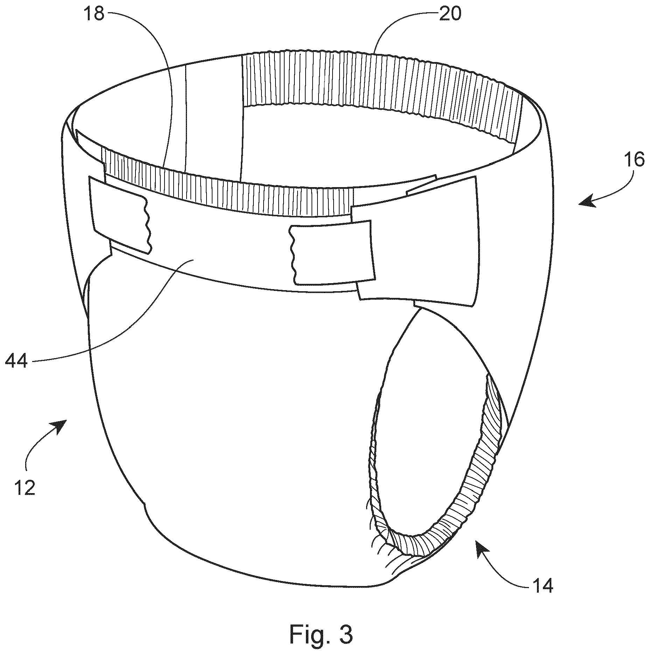

An example absorbent article 10 according to the present disclosure, shown in the form of a taped diaper, is represented in Figs. 1-3. Fig. 1 is a plan view of the example absorbent article 10, garment-facing surface 2 facing the viewer in a flat, laid-out state (i.e., no elastic contraction). Fig. 2 is a plan view of the example absorbent article 10 of Fig. 1, wearer-facing surface 4 facing the viewer in a flat, laid-out state. Fig. 3 is a front perspective view of the absorbent article 10 of Figs. 1 and 2 in a fastened configuration. The absorbent article 10 of Figs. 1-3 is shown for illustration purposes only as the present disclosure may be used for making a wide variety of diapers, including adult incontinence products, pants, or other absorbent articles, such as sanitary napkins and absorbent pads, for example.

The absorbent article 10 may comprise a front waist region 12, a crotch region 14, and a back waist region 16. The crotch region 14 may extend intermediate the front waist region 12 and the back waist region 16. The front wait region 12, the crotch region 14, and the back waist region 16 may each be 1/3 of the length of the absorbent article 10. The absorbent article 10 may comprise a front end edge 18, a back end edge 20 opposite to the front end edge 18, and longitudinally extending, transversely opposed side edges 22 and 24 defined by the chassis 52.

The absorbent article 10 may comprise a liquid permeable topsheet 26, a liquid impermeable backsheet 28, and an absorbent core 30 positioned at least partially intermediate the topsheet 26 and the backsheet 28. The absorbent article 10 may also comprise one or more pairs of barrier leg cuffs 32 with or without elastics 33, one or more pairs of leg elastics 34, one or more elastic waistbands 36, and/or one or more acquisition materials 38. The acquisition material or materials 38 may be positioned intermediate the topsheet 26 and the absorbent core 30. An outer cover material 40, such as a nonwoven material as disclosed herein, may cover a garment-facing side of the backsheet 28. The absorbent article 10 may comprise back ears 42 in the back waist region 16. The back ears 42 may comprise fasteners 46 and may extend from the back waist region 16 of the absorbent article 10 and attach (using the fasteners 46) to the landing zone area or landing zone material 44 on a garment-facing portion of the front waist region 12 of the absorbent article 10. The absorbent article 10 may also have front ears 47 in the front waist region 12. The absorbent article 10 may have a central lateral (or transverse) axis 48 and a central longitudinal axis 50. The central lateral axis 48 extends perpendicular to the central longitudinal axis 50.

In other instances, the absorbent article may be in the form of a pant having permanent or refastenable side seams. Suitable refastenable seams are disclosed in U.S. Pat. Appl. Pub. No. 2014/0005020 and U.S. Pat. No. 9,421,137.

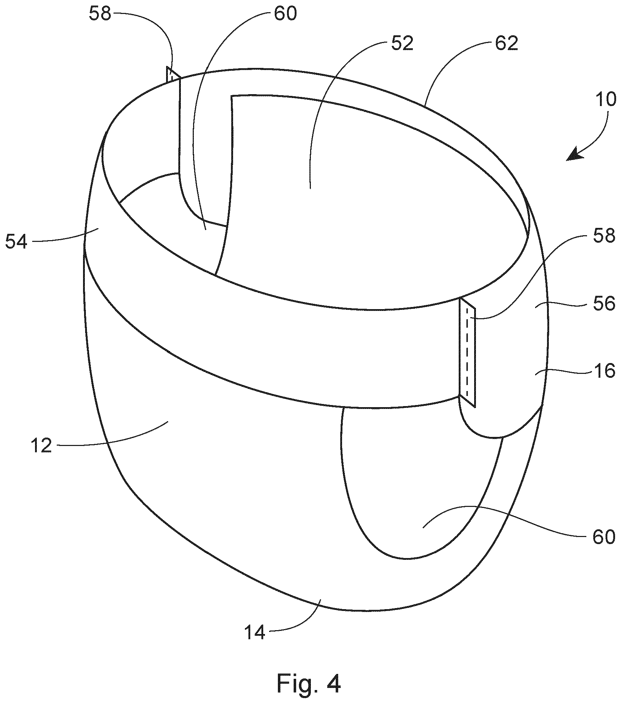

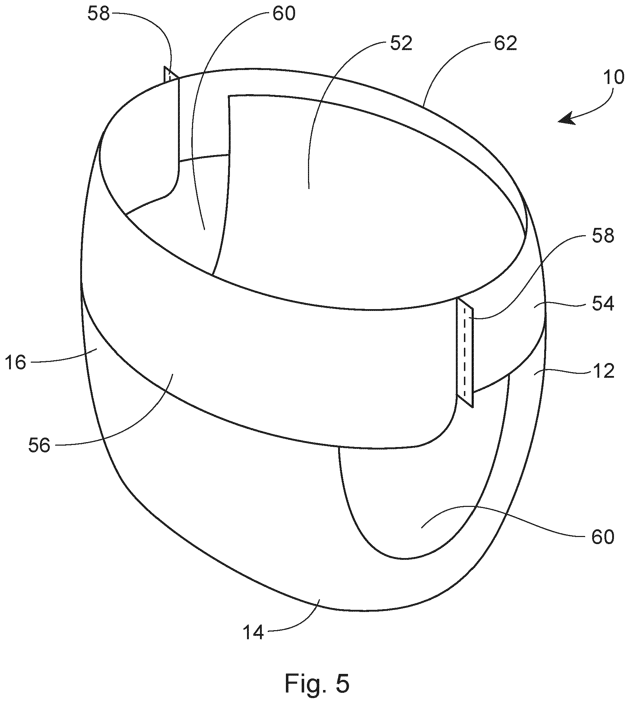

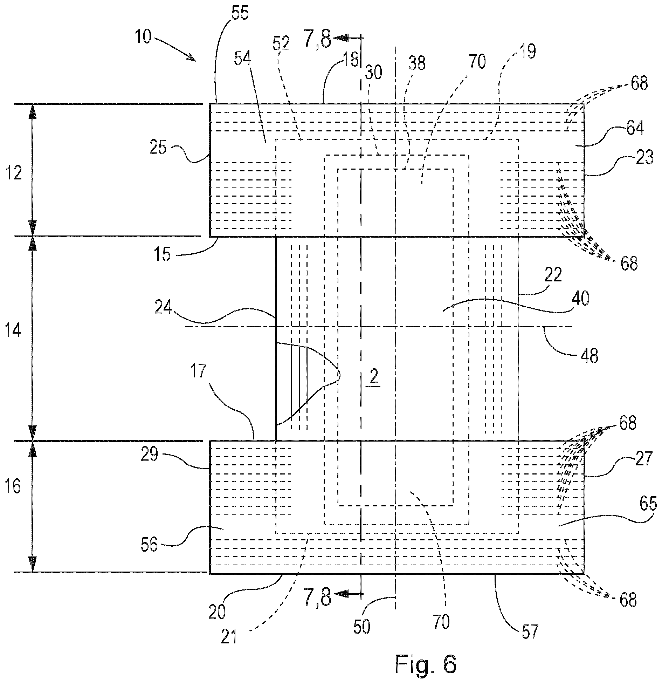

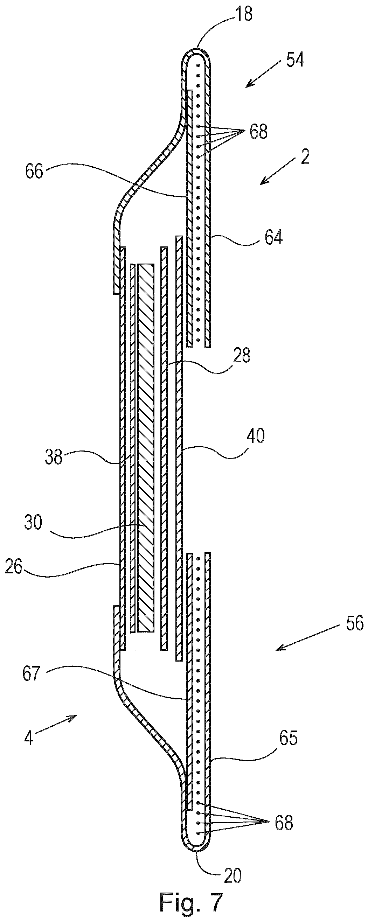

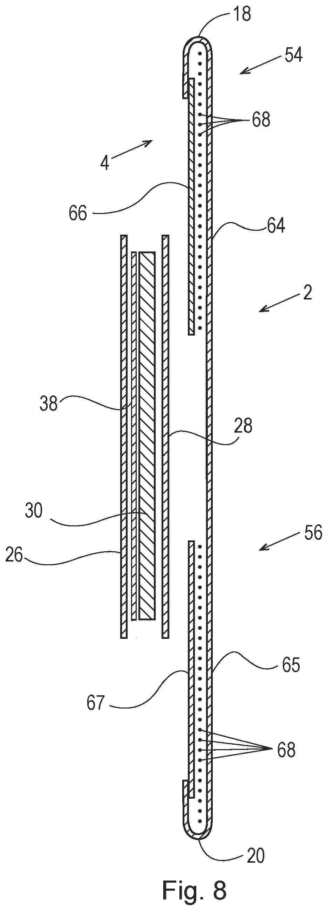

Referring to Figs. 4-8, an example absorbent article 10 in the form of a pant is illustrated. Fig. 4 is a front perspective view of the absorbent article 10. Fig. 5 is a rear perspective view of the absorbent article 10. Fig. 6 is a plan view of the absorbent article 10, laid flat, with the garment-facing surface facing the viewer. Elements of Fig. 4-8 having the same reference number as described above with respect to Figs. 1-3 may be the same element (e.g., absorbent core 30). Fig. 7 is an example cross-sectional view of the absorbent article taken about line 7-7 of Fig. 6. Fig. 8 is an example cross-sectional view of the absorbent article taken about line 8-8 of Fig. 6. Figs. 7 and 8 illustrate example forms of front and back belts 54, 56. The absorbent article 10 may have a front waist region 12, a crotch region 14, and a back waist region 16. Each of the regions 12, 14, and 16 may be 1/3 of the length of the absorbent article 10. The absorbent article 10 may have a chassis 52 (sometimes referred to as a central chassis or central panel) comprising a topsheet 26, a backsheet 28, and an absorbent core 30 disposed at least partially intermediate the topsheet 26 and the backsheet 28, and an optional acquisition material 38, similar to that as described above with respect to Figs. 1-3. The absorbent article 10 may comprise a front belt 54 in the front waist region 12 and a back belt 56 in the back waist region 16. The chassis 52 may be joined to a wearer-facing surface 4 of the front and back belts 54, 56 or to a garment-facing surface 2 of the belts 54, 56. Side edges 23 and 25 of the front belt 54 may be joined to side edges 27 and 29, respectively, of the back belt 56 to form two side seams 58. The side seams 58 may be any suitable seams known to those of skill in the art, such as butt seams or overlap seams, for example. When the side seams 58 are permanently formed or refastenably closed, the absorbent article 10 in the form of a pant has two leg openings 60 and a waist opening circumference 62. The side seams 58 may be permanently joined using adhesives or bonds, for example, or may be refastenably closed using hook and loop fasteners, for example.

Belts

Referring to Figs. 7 and 8, the front and back belts 54 and 56 may comprise front and back inner belt layers 66 and 67 and front and back outer belt layers 64 and 65 having an elastomeric material (e.g., strands 68 or a film (which may be apertured)) disposed at least partially therebetween. The elastic elements 68 or the film may be relaxed (including being cut) to reduce elastic strain over the absorbent core 30 or, may alternatively, run continuously across the absorbent core 30. The elastics elements 68 may have uniform or variable spacing therebetween in any portion of the belts. The elastic elements 68 may also be pre-strained the same amount or different amounts. The front and/or back belts 54 and 56 may have one or more elastic element free zones 70 where the chassis 52 overlaps the belts 54, 56. In other instances, at least some of the elastic elements 68 may extend continuously across the chassis 52.

The front and back inner belt layers 66, 67 and the front and back outer belt layers 64, 65 may be joined using adhesives, heat bonds, pressure bonds or thermoplastic bonds. Various suitable belt layer configurations can be found in U.S. Pat. No. 9,072,632.

Front and back belt end edges 55 and 57 may extend longitudinally beyond the front and back chassis end edges 19 and 21 (as shown in Fig. 6) or they may be co-terminus. The front and back belt side edges 23, 25, 27, and 29 may extend laterally beyond the chassis side edges 22 and 24. The front and back belts 54 and 56 may be continuous (i.e., having at least one layer that is continuous) from belt side edge to belt side edge (e.g., the transverse distances from 23 to 25 and from 27 to 29). Alternatively, the front and back belts 54 and 56 may be discontinuous from belt side edge to belt side edge (e.g., the transverse distances from 23 to 25 and 27 to 29), such that they are discrete.

As disclosed in U.S. Pat. No. 7,901,393, the longitudinal length (along the central longitudinal axis 50) of the back belt 56 may be greater than the longitudinal length of the front belt 54, and this may be particularly useful for increased buttocks coverage when the back belt 56 has a greater longitudinal length versus the front belt 54 adjacent to or immediately adjacent to the side seams 58.

The front outer belt layer 64 and the back outer belt layer 65 may be separated from each other, such that the layers are discrete or, alternatively, these layers may be continuous, such that a layer runs continuously from the front belt end edge 55 to the back belt end edge 57. This may also be true for the front and back inner belt layers 66 and 67 - that is, they may also be longitudinally discrete or continuous. Further, the front and back outer belt layers 64 and 65 may be longitudinally continuous while the front and back inner belt layers 66 and 67 are longitudinally discrete, such that a gap is formed between them - a gap between the front and back inner and outer belt layers 64, 65, 66, and 67 is shown in Fig. 7 and a gap between the front and back inner belt layers 66 and 67 is shown in Fig. 8. In some configurations shown in Fig. 7 and 8, portions of the outer belt layers 64, 65 may be folded over onto the inner belt layers, respectively. In addition, as shown in Fig. 7, portions of the outer belt layers 64, 65 may also be folded over onto the chassis 52.

The front and back belts 54 and 56 may include slits, holes, and/or perforations providing increased breathability, softness, and a garment-like texture. Underwear-like appearance can be enhanced by substantially aligning the waist and leg edges at the side seams 58 (see Figs. 4 and 5).

The front and back belts 54 and 56 may comprise graphics (see e.g., 78 of Fig. 1). The graphics may extend substantially around the entire circumference of the absorbent article 10 and may be disposed across side seams 58 and/or across proximal front and back belt edges 15 and 17; or, alternatively, adjacent to the seams 58 and/or proximal front and back belt edges 15 and 17 in the manner described in U.S. Pat. No. 9,498, 389 to create a more underwear-like article. The graphics may also be discontinuous.

Alternatively, instead of attaching belts 54 and 56 to the chassis 52 to form a pant, discrete side panels may be attached to side edges of the chassis 22 and 24. Suitable forms of pants comprising discrete side panels are disclosed in U.S. Pat. Nos. 6,645,190; 8,747,379; 8,372,052; 8,361,048; 6,761,711; 6,817,994; 8,007,485; 7,862,550; 6,969,377; 7,497,851; 6,849,067; 6,893,426; 6,953,452; 6,840,928; 8,579,876; 7,682,349; 7,156,833; and 7,201,744.

The nonwovens of the present disclosure may be used as nonwoven components of the belts, or portions thereof.

Topsheet

The topsheet 26 is the part of the absorbent article 10 that is in contact with the wearer's skin. The topsheet 26 may be joined to portions of the backsheet 28, the absorbent core 30, the barrier leg cuffs 32, and/or any other layers as is known to those of ordinary skill in the art. The topsheet 26 may be compliant, soft-feeling, and non-irritating to the wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers. The topsheet may have one or more layers. The topsheet may be apertured (Fig. 2, element 31), may have any suitable three-dimensional features, and/or may have a plurality of embossments (e.g., a bond pattern). The topsheet may comprise a variable basis weight nonwoven material, such as those described in U.S. Pat. Appl. No. 2017/0191198. The topsheet may be apertured by overbonding a material and then rupturing the overbonds through ring rolling, such as disclosed in U.S. Patent No. 5,628,097, to Benson et al., issued on May 13, 1997, and disclosed in U.S. Pat. Appl. Publication No. US 2016/0136014 to Arora et al. Any portion of the topsheet may be coated with a skin care composition, an antibacterial agent, a surfactant, and/or other beneficial agents. The topsheet may be hydrophilic or hydrophobic or may have hydrophilic and/or hydrophobic portions or layers. If the topsheet is hydrophobic, typically apertures will be present so that bodily exudates may pass through the topsheet. The topsheet may comprise a one or more layer hydroentangled material with or without apertures. The topsheet may comprise a variable basis weight nonwoven material.

The nonwovens of the present disclosure may be used as nonwoven components of the topsheet, or portions thereof.

Backsheet

The backsheet 28 is generally that portion of the absorbent article 10 positioned proximate to the garment-facing surface of the absorbent core 30. The backsheet 28 may be joined to portions of the topsheet 26, the outer cover material 40, the absorbent core 30, and/or any other layers of the absorbent article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials, such as films, which permit vapors to escape from the absorbent article, while still preventing, or at least inhibiting, bodily exudates from passing through the backsheet. The backsheet 28 may be coterminous with the outer cover material 40.

Outer Cover Material

The outer cover material (sometimes referred to as a backsheet nonwoven) 40 may comprise one or more nonwoven materials joined to the backsheet 28 and that covers the backsheet 28. The outer cover material 40 forms at least a portion of the garment-facing surface 2 of the absorbent article 10 and effectively "covers" the backsheet 28 so that film is not present on the garment-facing surface 2. The outer cover material 40 may comprise a bond pattern, apertures, and/or three-dimensional features. The outer cover material 40 may be a hydroentangled nonwoven material or a variable basis weight nonwoven material. Such a material may have one or more layers. The nonwovens of the present disclosure may be used as outer cover nonwoven material, or portions thereof.

Absorbent Core

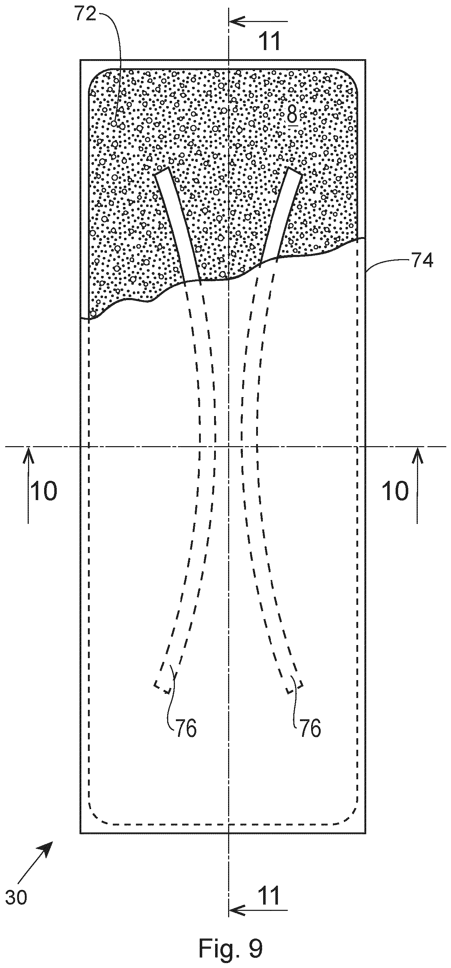

As used herein, the term "absorbent core" refers to a component of the absorbent article 10 for absorbing and containing liquid such as urine received by the absorbent article. The absorbent core thus typically has a high absorbent capacity. An example absorbent core 30 is schematically shown in Figs. 9-11. The absorbent core comprises an absorbent material 72. The absorbent typically comprises a core wrap 74 that encloses or sandwiches the absorbent material.

The core wrap may be a single material that is folded and attached to itself, or it may comprise a separate top layer and bottom layer that may be bonded, adhesively joined, or otherwise joined together. The top and bottom layers of the core wrap may be the same or different. The absorbent material typically comprises superabsorbent particles which are optionally mixed with cellulose fibers. As used herein, "absorbent core" does not include any acquisition-distribution systems, topsheet, or backsheet of the absorbent article.

The example absorbent core 30 shown in isolation in Figs. 9-11 is in the dry state (before use). The absorbent core may typically have a generally rectangular shape as defined by its longitudinal edges and transversal front edge and back edge or may have other shapes.

Absorbent material 72 may be deposited as an absorbent layer having a generally rectangular outline, as represented in Fig. 9. A wide variety of absorbent cores may also be used. The absorbent material 72 layer may also have a non-rectangular perimeter ("shaped" core), in particular, the absorbent material 72 may define a tapering along its width towards the central region of the core (or "dog-bone" shape). In this way, the absorbent material deposition area may have a relatively narrow width in an area of the core intended to be placed in the crotch region of the absorbent article. This may provide for example better wearing comfort. Other shapes can also be used such as a "T" or "Y" or "hourglass" for the area of the absorbent material.

The absorbent material 72 may be any conventional absorbent material known in the art. For example, the absorbent material may comprise a blend of cellulose fibers and superabsorbent particles ("SAP"), typically with the percentage of SAP ranging from about 50% to about 75% by weight of the absorbent material. The absorbent material may also be free of cellulose fibers, as is known in so-called airfelt-free cores, where the absorbent material consists, or consists essentially, of SAP. The absorbent material may also be a high internal phase emulsion foam. High loft nonwoven materials may be present in the core bags, or proximate but outside the core bags. The absorbent material may comprise one or more layers of a coform material. The coform material may comprise a mixture of fibers and an absorbent material. The fibers may comprise staple fibers such as synthetic fibers or absorbent fibers. The synthetic fibers may comprise polypropylene fibers, polyethylene fibers, and/or bicomponent fibers. The absorbent fibers may comprise pulp, lyocell, and/or viscose. The fibers may also comprise thermoplastic filaments (scattered or interconnected networks). The absorbent material may comprise a super absorbent polymeric material in fiber or particle form. The one or more layers of coform material may comprise virgin or recycled materials. The staple fibers may be present in the coform material in an amount of from about 5wt. % to about 50wt.%. The absorbent material may be present in the nonwoven web in an amount of from about 50wt.% to about 95wt.%. The staple fibers may have an average length of from about 5 mm to about 50 mm. The staple fibers and absorbent fibers may be thermally bonded or hydraulically entangled to form the nonwoven web (as disclosed in WO2023/022979).

"Superabsorbent polymer" or "SAP" refers herein to absorbent materials, typically cross-linked polymeric materials, that can absorb at least 10 times their weight of an aqueous 0.9% saline solution as measured using the Centrifuge Retention Capacity (CRC) test (EDANA method WSP 241.2.R3 (12)). The SAP may in particular have a CRC value of at least 20 g/g, in particular of from about 20 g/g to about 40 g/g. "Superabsorbent polymer particles", as used herein, refers to a superabsorbent polymer material which is in particulate form so as to be flowable in the dry state.

Various absorbent core designs comprising high amounts of SAP have been proposed in the past, see for example in U.S. Pat. No. 5,599,335 (Goldman), EP1,447,066 (Busam), WO95/11652 (Tanzer), U.S. Pat. Appl. Pub. No. 2008/0312622A1 (Hundorf), WO2012/052172 (Van Malderen). In particular, the SAP printing technology as disclosed in U.S. Pat. No. 7,838,722 (Blessing), U.S. Pat. No. 9,072,634 and U.S. Pat. No. 8,206,533 (both to Hundorf et al.) may be used. The present disclosure however is not limited to a particular type of absorbent core. The absorbent core may also comprise one or more glues such as an auxiliary glue applied between the internal surface of one (or both) of the core wrap layers and the absorbent material to reduce leakage of SAP outside the core wrap. A micro-fibrous adhesive net may also be used in air-felt free cores as described in the above Hundorf references. These glues are not represented in the Figures for simplicity. Other core constructions comprising a high loft nonwoven substrate, such as a carded nonwoven layer, having a porous structure into which SAP particles have been deposited, may also be used in present disclosure.

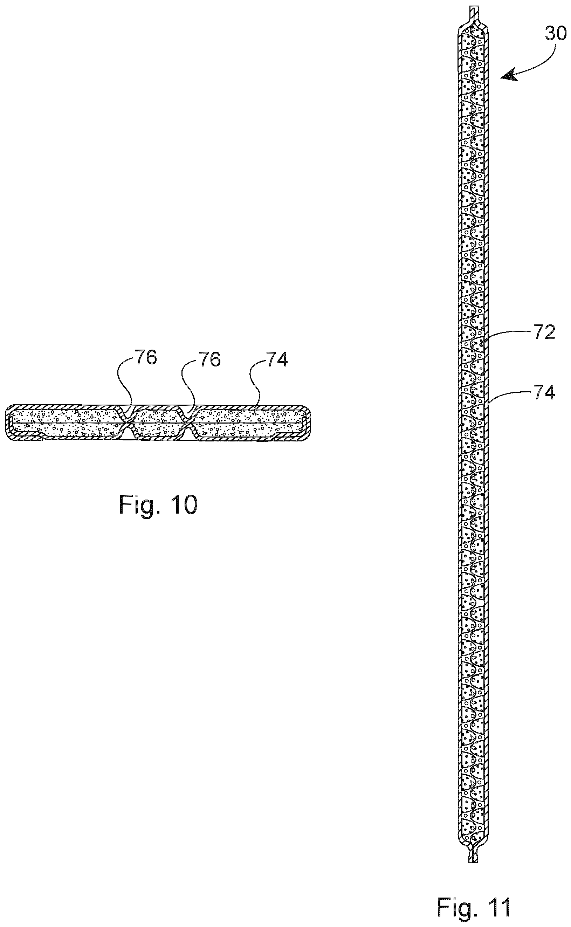

The absorbent material may be deposited as a continuous layer within the core wrap. The absorbent material may also be present discontinuously, for example, as individual pockets or stripes of absorbent material enclosed within the core wrap and separated from each other by material-free junction areas. A continuous layer of absorbent material, in particular of SAP, may also be obtained by combining two absorbent layers having matching discontinuous absorbent material application pattern, wherein the resulting layer is substantially continuously distributed across the absorbent particulate polymer material area, as illustrated in Figs. 10-11. As for example taught in U.S. Pat. Appl. Pub. No. 2008/0312622A1 (Hundorf), each absorbent material layer may thus comprise a pattern having absorbent material land areas and absorbent material-free junction areas, wherein the absorbent material land areas of the first layer correspond substantially to the absorbent material-free junction areas of the second layer and vice versa.

The basis weight (amount deposited per unit of surface) of the absorbent material may also be varied to create a profiled distribution of absorbent material, in particular in the longitudinal direction to provide more absorbency towards the center and the middle of the core, but also in the transversal direction, or both directions of the core. The absorbent core may also comprise one or more longitudinally (or otherwise) extending channels 76, which are areas of the absorbent layer substantially free of absorbent material within the absorbent material layer. The top side of the core wrap may be advantageously bonded to the bottom side of the core by adhesive, mechanical or ultrasonic bonding through these material-free areas. Example disclosures of such channels in an airfelt-free core can be found in U.S. Pat. No. 9,789,011. One or more channels may also be formed in absorbent cores comprising a mix of cellulose fibers and SAP particles. These channels may embody any suitable shapes and any suitable number of channels may be provided. In other instances, the absorbent core may be embossed to create the impression of channels, with absorbent material remaining within the channels. The absorbent core in Figs. 9-11 is merely an example absorbent core. Many other absorbent cores with or without channels are also within the scope of the present disclosure.

Barrier Leg Cuffs/Leg Elastics

Referring to Figs. 1 and 2, for example, the absorbent article 10 may comprise one or more pairs of barrier leg cuffs 32 and one or more pairs of leg elastics 34. The barrier leg cuffs 32 may be positioned laterally inboard of leg elastics 34. Each barrier leg cuff 32 may be formed by a piece of material which is bonded to the absorbent article 10 so it can extend upwards from a wearer-facing surface 4 of the absorbent article 10 and provide improved containment of body exudates approximately at the junction of the torso and legs of the wearer. The barrier leg cuffs 32 are delimited by a proximal edge joined directly or indirectly to the topsheet and/or the backsheet and a free terminal edge, which is intended to contact and form a seal with the wearer's skin. The barrier leg cuffs 32 may extend at least partially between the front end edge 18 and the back end edge 20 of the absorbent article 10 on opposite sides of the central longitudinal axis 50 and may be at least present in the crotch region 14. The barrier leg cuffs 32 may each comprise one or more elastics 33 (e.g., elastic strands or strips) near or at the free terminal edge. These elastics 33 cause the barrier leg cuffs 32 to help form a seal around the legs and torso of a wearer. The leg elastics 34 extend at least partially between the front end edge 18 and the back end edge 20. The leg elastics 34 essentially cause portions of the absorbent article 10 proximate to the chassis side edges 22, 24 to help form a seal around the legs of the wearer. The leg elastics 34 may extend at least within the crotch region 14. The barrier leg cuffs 32 may comprise one or more layers of nonwoven material. The nonwoven material may be hydroentangled.

The nonwovens of the present disclosure may be used as nonwoven components of the barrier leg cuffs, or portions thereof.

Elastic Waistband

Referring to Figs. 1 and 2, the absorbent article 10 may comprise one or more elastic waistbands 36. The elastic waistbands 36 may be positioned on the garment-facing surface 2 or the wearer-facing surface 4. Alternatively, the elastic waistbands may be positioned intermediate the topsheet and the backsheet. As an example, a first elastic waistband 36 may be present in the front waist region 12 near the front end edge 18 and a second elastic waistband 36 may be present in the back waist region 16 near the back end edge 20. The elastic waistbands 36 may aid in sealing the absorbent article 10 around a waist of a wearer and at least inhibiting bodily exudates from escaping the absorbent article 10 through the waist opening circumference. In some instances, an elastic waistband may fully surround the waist opening circumference of an absorbent article. The elastic waistband may comprise an elastic film joined to the topsheet and a nonwoven material covering the elastic film. In other instances, the elastic waistband may comprise an elastic film sandwiched between two nonwoven materials. The elastic film may be ultrasonically bonded, or otherwise bonded or attached, to the one or more nonwoven materials. The one or more nonwoven materials may be hydroentangled. The elastic film and/or the nonwoven materials may be preactivated (i.e., activated prior to being joined together) or the formed elastic film/nonwoven laminate may be activated post laminate formation.

The nonwovens of the present disclosure may be used as nonwoven components of the waistband, or portions thereof.

Acquisition Materials

Referring to Figs. 1, 2, 7, and 8, one or more acquisition materials 38 may be present at least partially intermediate the topsheet 26 and the absorbent core 30. The acquisition materials 38 are typically hydrophilic materials that provide significant wicking of bodily exudates. These materials may dewater the topsheet 26 and quickly move bodily exudates into the absorbent core 30. The acquisition materials 38 may comprise one or more nonwoven materials, foams, formed films, apertured formed films, cellulosic materials, cross-linked cellulosic materials, air laid cellulosic nonwoven materials, spunlace materials, or combinations thereof, for example. The acquisition material may be rectangular or may be shaped, such as hourglass shaped. Typically, an acquisition material 38 may have a width and length that are smaller than the width and length of the topsheet 26. The acquisition material may be a secondary topsheet in the feminine pad context. The acquisition materials may have one or more channels as described above with reference to the absorbent core 30 (including the embossed version). The channels in the acquisition material may align or not align with channels in the absorbent core 30. In an example, a first acquisition material may comprise a nonwoven material and as second acquisition material may comprise a cross-linked cellulosic material.

The nonwovens of the present disclosure may be used as nonwoven components of the acquisition material, or portions thereof.

Landing Zone

Referring to Figs. 1 and 2, the absorbent article 10 may have a landing zone area 44 that is formed in a portion of the garment-facing surface 2 of the outer cover material 40. The landing zone area 44 may be in the back waist region 16 if the absorbent article 10 fastens from front to back or may be in the front waist region 12 if the absorbent article 10 fastens back to front. In some instances, the landing zone 44 may be or may comprise one or more discrete nonwoven materials that are attached to a portion of the outer cover material 40 in the front waist region 12 or the back waist region 16 depending upon whether the absorbent article fastens in the front or the back. In essence, the landing zone 44 is configured to receive the fasteners 46 and may comprise, for example, a plurality of loops configured to be engaged with, a plurality of hooks on the fasteners 46, or *vice versa.*

The nonwovens of the present disclosure may be used as nonwoven components of the landing zone, or portions thereof.

Wetness Indicator/Graphics

Referring to Fig. 1, the absorbent articles 10 of the present disclosure may comprise graphics 78 and/or wetness indicators 80 that are visible from the garment-facing surface 2. The graphics 78 may be printed on the landing zone 40, the backsheet 28, and/or at other locations. The wetness indicators 80 are typically applied to the absorbent core facing side of the backsheet 28, so that they can be contacted by bodily exudates within the absorbent core 30. In some instances, the wetness indicators 80 may form portions of the graphics 78. For example, a wetness indicator may appear or disappear and create/remove a character within some graphics. In other instances, the wetness indicators 80 may coordinate (e.g., same design, same pattern, same color) or not coordinate with the graphics 78.

Front and Back Ears

Referring to Figs. 1 and 2, as referenced above, the absorbent article 10 may have front and/or back ears 47, 42 in a taped diaper context. Only one set of ears may be required in most taped diapers. The single set of ears may comprise fasteners 46 configured to engage the landing zone or landing zone area 44. If two sets of ears are provided, in most instances, only one set of the ears may have fasteners 46, with the other set being free of fasteners. Any ears with fasteners may be configured to engage an opposing ear or the landing zone or landing zone area 44. One set of ears may comprise primary fasteners and the other set of ears may comprise secondary fasteners. The ears, or portions thereof, may be elastic or may have elastic panels. In an example, an elastic film or elastic strands may be positioned intermediate a first nonwoven material and a second nonwoven material. One or more of the first and second nonwoven materials may be hydroentangled. The elastic film may or may not be apertured. The ears may be shaped. The ears may be integral (e.g., extension of the outer cover material 40, the backsheet 28, and/or the topsheet 26) or may be discrete components attached to a chassis 52 of the absorbent article on a wearer-facing surface 4, on the garment-facing surface 2, or intermediate the two surfaces 4, 2. The back ears may comprise an elastic film sandwiched between two nonwoven materials forming a laminate. The elastic film and/or the nonwoven materials may be pre-activated (i.e., activated prior to being joined together) and the laminate may be joined by ultrasonic bonds. Such laminates are disclosed in U.S. Pat. No. 10,485,713. In other instances, the elastic film may not be preactivated and instead the laminate may be activated after the laminate is formed. Such laminates may also be ultrasonically bonded.

The nonwovens of the present disclosure may be used as nonwoven components of the front and back ears, or portions thereof.

Masking Layer

One or more masking layers or materials may be provided in the absorbent articles 10. A masking layer may be a layer that provides a cushiony feel when the absorbent article is touched from the garment-facing surface 2 or the wearer-facing surface 4. The masking layer may "mask" a grainy feel potentially caused by the absorbent material 72, such as superabsorbent polymers. The masking layer may "mask" bodily exudates from being visible when viewing the wearer-facing surface 4 or the garment-facing surface 2 of the absorbent article 10. The masking layer may have a basis weight in the range of about 15 gsm to about 50 gsm or about 15 gsm to about 40 gsm. The masking layer may comprise one or more nonwoven materials (e.g., a hydroentangled nonwoven material), foams, pulp layers, and/or other suitable materials. The masking layer may be the outer cover material 40. The masking layer may be the layer forming the garment-facing side or the wearer-facing side of the core bag 74. The masking layer may be a separate material positioned intermediate the garment-facing side of the core bag 74 and the liquid impermeable backsheet 28.

The nonwovens of the present disclsoure may be used as nonwoven components of the masking layer, or portions thereof.

Packages

The absorbent articles of the present disclosure may be placed into packages. The packages may comprise polymeric films, paper, and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages. The packages may comprise polymeric films comprising recycled material, such as about 20% to about 100%, about 30% to about 90%, about 30% to about 80%, about 40% to about 60%, or about 50% recycled material. The recycled material may comprise post-industrial recycled material (PIR) and/or post-consumer recycled material (PCR). In some instances, the polymeric films used for the packages may comprise two outer layers and one or more inner layers. The one or more inner layers may comprise the recycled material or may comprise more recycled material than the outer layers. The recycled material may comprise recycled polyethylene. The recycled material may comprise recycled polyethylene PIR from trim from the packaging operation.

The package material may comprise paper, paper based material, paper with one or more barrier layers, or a paper/film laminate. The package material may be in the range of about 50 gsm to about 100 gsm or about 70 gsm to about 90 gsm and the one or more barrier layers may be in the range of about 3 gsm to about 15 gsm. The paper based package material with or without one or more barrier layers may exhibit a machine direction tensile strength of at least 5.0 kN/m, a machine direction stretch of at least 3 percent, a cross-machine direction tensile strength of at least 3 kN/m, and a cross-direction stretch at break of at least 4 percent, each as determined via ISO 1924-3.

The paper based package material or paper based package material comprising a barrier layer or film may be recyclable or recyclable in normal paper recycling operations. The recyclability extent of the paper based package may be determined via recyclable percentage. The paper based package of the present disclosure may exhibit recyclable percentages of 70 percent or greater, 80 percent or greater, or 90 percent or greater. The paper based package of the present disclosure may have a recyclable percentage of between 70 percent to about 99.9 percent, between about 80 percent to about 99.9 percent, or between about 90 percent to about 99.9 percent. In one example, the package material of the present disclosure may exhibit a recyclable percentage of from about 95 percent to about 99.9 percent, from about 97 percent to about 99.9 percent, or from about 98 percent to about 99.9 percent. The recyclable percentage of the paper based package may be determined via test PTS-RH:021/97 (Draft Oct. 2019) under category II, as performed by Papiertechnische Stiftung located at Pirnaer Strasse 37, 01809 Heidenau, Germany. In another instance, the paper based packages of the present disclosure may exhibit an overall "pass" test outcome as determined by PTS-RH:021/97 (Draft Oct. 2019) under category II method. Any of the paper based packages may have opening features, such as lines of perforation, and may also have handles.

Arrays

"Array" means a display of packages comprising disposable absorbent articles of different article constructions (e.g., different elastomeric materials [compositionally and/or structurally] in the side panels, back ears, side flaps and/or belts flaps, different graphic elements, different product structures, fasteners, waistbands, or lack thereof). The packages may have the same brand and/or sub-brand and/or the same trademark registration and/or having been manufactured by or for a common manufacturer and the packages may be available at a common point of sale (e.g. oriented in proximity to each other in a given area of a retail store). An array is marketed as a line-up of products normally having like packaging elements (e.g., packaging material type, film, paper, dominant color, design theme, etc.) that convey to consumers that the different individual packages are part of a larger line-up. Arrays often have the same brand, for example, "Huggies," and same sub-brand, for example, "Pull-Ups." A different product in the array may have the same brand "Huggies" and the sub-brand "Little Movers." The differences between the "Pull-Ups" product of the array and the "Little Movers" product in the array may include product form, application style, different fastening designs or other structural elements intended to address the differences in physiological or psychological development. Furthermore, the packaging is distinctly different in that "Pull-Ups" is packaged in a predominately blue or pink film bag and "Little Movers" is packaged in a predominately red film bag.

Further regarding "Arrays," as another example an array may be formed by different products having different product forms manufactured by the same manufacturer, for example, "Kimberly-Clark", and bearing a common trademark registration for example, one product may have the brand name "Huggies," and sub-brand, for example, "Pull-Ups." A different product in the array may have a brand/sub-brand "Good Nites" and both are registered trademarks of The Kimberly-Clark Corporation and/or are manufactured by Kimberly-Clark. Arrays also often have the same trademarks, including trademarks of the brand, sub-brand, and/or features and/or benefits across the line-up. "On-line Array" means an "Array" distributed by a common on-line source.

Sanitary Napkin

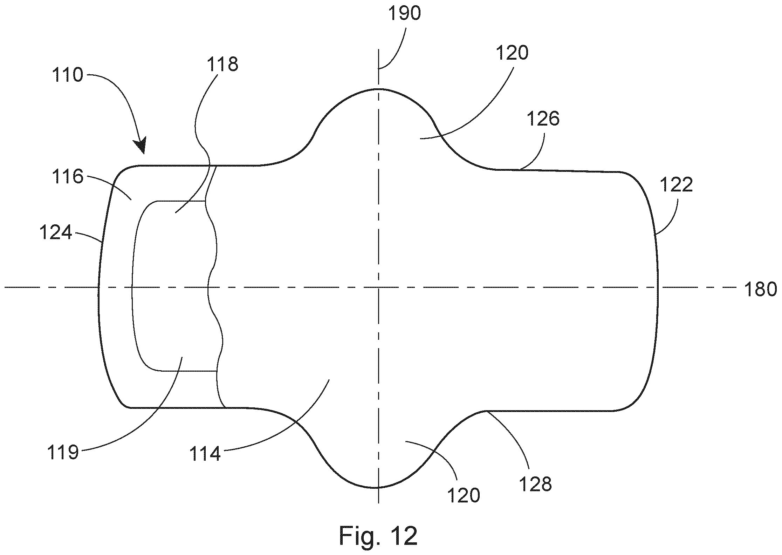

Referring to Fig. 12, an absorbent article of the present disclosure may be a sanitary napkin 110. The sanitary napkin 110 may comprise a liquid permeable topsheet 114, a liquid impermeable, or substantially liquid impermeable, backsheet 116, and an absorbent core 118. The liquid impermeable backsheet 116 may or may not be vapor permeable. The absorbent core 118 may have any or all of the features described herein with respect to the absorbent core 30 and, in some forms, may have a secondary topsheet 119 (STS) instead of the acquisition materials disclosed above. The STS 119 may comprise one or more channels, as described above (including the embossed version). In some forms, channels in the STS 119 may be aligned with channels in the absorbent core 118. The sanitary napkin 110 may also comprise wings 120 extending outwardly with respect to a longitudinal axis 180 of the sanitary napkin 110. The sanitary napkin 110 may also comprise a lateral axis 190. The wings 120 may be joined to the topsheet 114, the backsheet 116, and/or the absorbent core 118. The sanitary napkin 110 may also comprise a front edge 122, a back edge 124 longitudinally opposing the front edge 122, a first side edge 126, and a second side edge 128 longitudinally opposing the first side edge 126. The longitudinal axis 180 may extend from a midpoint of the front edge 122 to a midpoint of the back edge 124. The lateral axis 190 may extend from a midpoint of the first side edge 128 to a midpoint of the second side edge 128. The sanitary napkin 110 may also be provided with additional features commonly found in sanitary napkins as is known in the art.

The nonwovens of the present disclosure may be used as nonwoven components of the sanitary napkins, or portions thereof.

Fasteners

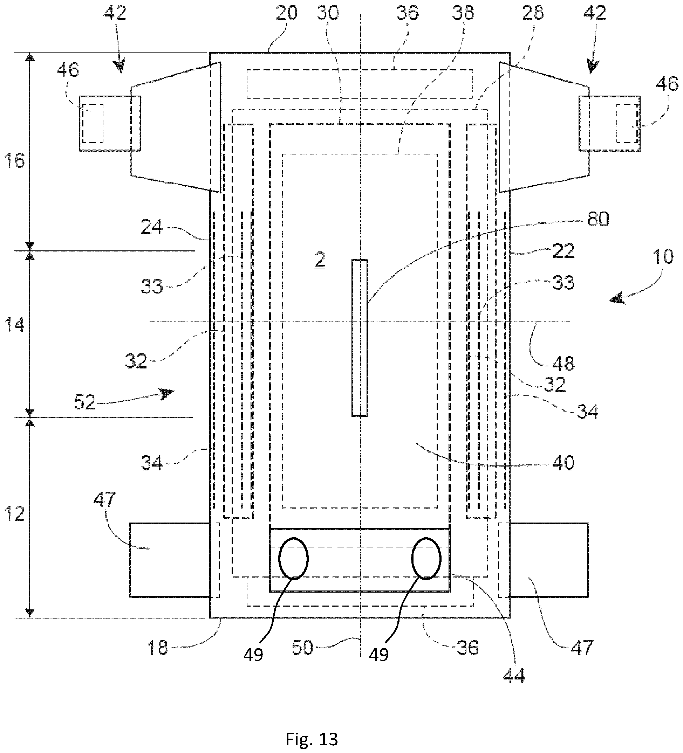

Referring to Fig. 13, the back ears 42 may comprise fasteners 46. The fasteners 46 may be configured to cooperate with the landing zone area or landing zone material 44 on the garment-facing surface of the front waist region 12 of the absorbent article 10. Additionally, the absorbent article may comprise one or more secondary fasteners 49. The secondary fasteners 49 may be disposed on the outer cover material 40 or a component of the absorbent article, such as the landing zone 44 as illustrated in Fig. 13. The secondary fasteners may be a separate material joined to the absorbent article. The secondary fasteners may be integrally formed from a material of the absorbent article. For example, the secondary fasteners may comprise one or more hooks or protrusions formed from the material of the outer cover material 40, the landing zone material 44, or a film. The hooks or protrusions may be formed using the process described in U.S. Pat. No. 8,784,722 to Rocha et al. The secondary fasteners may be configured to cooperate with a portion of the back ears. For example, a secondary fastener comprising hooks or protrusions are configured to engage the nonwoven material of the back ear. It is to be appreciated that the secondary fasteners may be any mechanical fastener that is configured to cooperate with a component of the absorbent article.

Bio-Based Content for Components

Components of the absorbent articles described herein may at least partially be comprised of bio-based content as described in U.S. Pat. Appl. No. 2007/0219521A1. For example, the superabsorbent polymer component may be bio-based via their derivation from bio-based acrylic acid. Bio-based acrylic acid and methods of production are further described in U.S. Pat. Appl. Pub. No. 2007/0219521 and U.S. Pat. Nos. 8,703,450; 9,630,901 and 9,822,197. Other components, for example nonwoven and film components, may comprise bio-based polyolefin materials. Bio-based polyolefins are further discussed in U.S. Pat. Appl. Pub. Nos. 2011/0139657, 2011/0139658, 2011/0152812, and U.S. Pat. Nos. 10,166,312 and 9,169,366. Example bio-based polyolefins for use in the present disclosure comprise polymers available under the designations SHA7260™, SHE150™, or SGM9450F™ (all available from Braskem S.A.).

An absorbent article component may comprise a bio-based content value from about 10% to about 100%, from about 25% to about 100%, from about 40% to about 100%, from about 50% to about 100%, from about 75% to about 100%, or from about 90% to about 100%, for example, using ASTM D6866-10, method B.

Recycle Friendly and Bio-Based Absorbent Articles

Components of the absorbent articles described herein may be recycled for other uses, whether they are formed, at least in part, from recyclable materials. Examples of absorbent article materials that may be recycled are nonwovens, films, fluff pulp, and superabsorbent polymers. The recycling process may use an autoclave for sterilizing the absorbent articles, after which the absorbent articles may be shredded and separated into different byproduct streams. Example byproduct streams may comprise plastic, superabsorbent polymer, and cellulose fiber, such as pulp. These byproduct streams may be used in the production of fertilizers, plastic articles of manufacture, paper products, viscose, construction materials, absorbent pads for pets or on hospital beds, and/or for other uses. Further details regarding absorbent articles that aid in recycling, designs of recycle friendly diapers, and designs of recycle friendly and bio-based component diapers, are disclosed in U.S. Pat. No. 11,433,158, issued on September 6, 2022.

EXAMPLES

Example 1

Polypropylene (PP) PCR waste was obtained from carpets. The PP waste was subjected to a solvent recycling process:
The sample of PCR polypropylene flake was processed using the following procedure:

1. 173 g of mixed color flake was loaded into a Parr Instrument Company Model 4552M 7.57 liter autoclave equipped with an overhead mechanical stirrer.
2. The autoclave was then completely filled with n-butane and equilibrated to an internal fluid temperature of 140°C and a fluid pressure of 900 psig (6.21 MPa).
3. The material in the autoclave was then extracted using the following procedure:

    a. The system was stirred for 10 min at 140°C and 900 psig (6.21 MPa).
    b. After stirring, the system was settled for 10 min at 140°C and 900 psig (6.21 MPa).
    c. One vessel volume of n-butane was flushed through the autoclave into a sample collection flask at 140°C and 900 psig (6.21 MPa).
    d. The above extraction procedure was repeated four more times.
    e. The samples collected for each extraction cycle were sequentially labeled "Fraction 1" through "Fraction 5".

4. The material remaining in the autoclave after extraction was then dissolved in n-butane using the following procedure:

    a. The system pressure was equilibrated to 1800 psig (12.41 MPa).
    b. The system was stirred for 10 min at 140°C and 1800 psig (12.41 MPa).
    c. The stirring was then stopped and the solution was allowed settle for 60 min at 140°C and 1800 psig (6.21 MPa).
    d. After settling, the solution was removed of the autoclave by displacement with pressurized n-butane (pre-equilibrated to 140°C and 1800 psig). The solution exited the autoclave through a dip tube was then passed through two heat-traced solid media columns. Each column had an ID of 0.68" (1.73 cm) and a length of about 9.5" (24.13 cm). In this example, both columns were empty and did not contain any solid media. The fluid stream leaving the bottom of the second pressure vessel was depressurized across an expansion valve into a side-arm Erlenmeyer flask. After depressurizing the fluid stream into the Erlenmeyer flask, the solvent vapor was vented through the side-arm port and any liquids/solids collected as fractions in flasks. Each fraction contained about 30 g of material and were labelled sequentially starting with "Fraction 6". Fractions were collected until no more material was observed eluting into the flask.

5. After all samples were collected, the autoclaved was equilibrated to atmospheric pressure and room temperature. All residual material in the autoclave was then collected as a residuum sample.

After solvent recycling a PP resin R1 having 100% TCC derived from waste carbon with low contaminants levels was obtained.

Table 1

| Substance | Carpet PCR PP Waste | PP Resin R1 after solvent recycling process |
|---|---|---|
| iso-Nonylphenolhexaethoxylate [$\mu$g/kg] | 7800 | <50 |
| iso-Nonylphenol [$\mu$g/kg] | 820 | <50 |
| OCDD [ng/kg] | 0.52 | <0.2 |
| Di-2-ethylhexyl phthalate [$\mu$g/kg] | 580 | <50 |
| Napthalene [$\mu$g/kg] | 11 | <1 |

The Melt Flow Rate (MFR) of the obtained recycled PP resin R1 was determined and found to be too low for spinning a nonwoven. The recycled PP resin R1 was treated with 1.2% by weight of Polyvel CR5 for vis-breaking and produced in a first precompounding step using a twin-screw extruder to produce RV1. Polyvel CR5 consists of PP and 5% by weight of peroxide (as available from Polyvel in August 2024). The amount of CR5 needed for the nonwoven making process was determined by adding different amounts and measuring the MFR and the Apparent Shear Viscosity needed to produce a 44 gsm nonwoven, for which the MD and CD strengths were determined. The process used to produce the 44gsm nonwoven was a standard spunbond process that consisted of an extruder, melt pump and fiber attenuation process, followed by laydown on a porous belt that is first bonded with a flat heated roll and later by a heated pattern roll. For comparison, the MFR and the Apparent Shear Viscosity was determined for a virgin PP resin (Exxon PP 3155E5 as available from ExxonMobil in August 2024) were determined and used as the reference for this example.

Table 2

| Resin | MFR (g/10min) resin | Apparent Shear Viscosity resin (Pa s; 230°C @ 500/s) | MD / CD strength of 44gsm nonwoven [N] |
|---|---|---|---|
| R1 | 7 | 180 | Not possible to produce |
| RV1 | 38 | 75 | 93/53 |
| Virgin PP resin (Comparative example) | 35 | 80 | 92 / 48 |

Net, a nonwoven with >98% of the TCC derived from waste carbon was obtained, which exhibited low levels of contaminants and parity mechanical properties to a nonwoven spun from virgin resin.

The nonwoven made from the RV1 resin and the virgin polypropylene resin of Table 2 may be tested in the Odor Analysis Test Method described herein. Both nonwovens may exhibit an odor intensity score of 0, less than 1, or less than 2. The inventive nonwoven may display this odor intensity score if its transformation from a recycle fiber starting point comprises the elimination of trace chemicals, absence of any purification solvents or byproducts, and an absence of odiferous compounds potentially introduced by visbreaking or the transformations involved in melt spinning. A nonwoven made of recycled materials with trace chemicals at high levels may have an odor grade of 3 or greater.

TEST METHODS

Trace Chemicals Test Method

The content on a mass per mass basis of trace chemicals present in the nonwoven or resin is determined using methods containing sample extraction and instrumental analysis approaches known of those of skill in the art and understood to be capable of extracting and detecting essentially all of a particular trace chemical in nonwovens or resins. Suitable overall method specifics vary based on the particular trace chemical being quantified, though a common overall method structure is generally followed. A suitable method generally begins with increasing the surface area of a nonwoven or resin sample by cutting, shredding, and/or milling into small particles. An extraction step is then performed using a solvent capable of dissolving or diffusing into the polymer sample and in which the trace chemical is readily soluble. The extraction process conditions (e.g., time and temperature) are optimized to maximize trace chemical recovery and common approaches include but are not limited to Soxhlet extraction, accelerated solvent extraction, and/or ultrasonic extraction. Internal standards (usually stable-isotope-labeled versions of the trace chemicals if mass spectrometry-based detection approaches are used) are added during the extraction step to account for potential losses during extraction and subsequent processing steps. After extraction is complete, the extraction solvent is isolated and worked up for instrumental analysis. Depending on the solvent used for extraction, the required quantitation limit, and the specific instrumental approach used, generation of a specimen suitable for instrumental analysis may require multiple cleanup and/or concentration steps including but not limited to centrifugation/filtration, liquid/liquid extraction, and/or column chromatography. The trace chemical in the worked-up specimen is then quantified using instrumental methods (e.g., GC-MS, GC-MS/MS, GC-HRMS, LC-MS/MS, etc.) known to those of skill in the art and specifically adapted to the trace chemical of interest. Suitable instrumental approaches may be developed de novo or may be based on standard, known methods for particular trace chemicals (e.g., US EPA Method 1613 in the case of dioxins and furans or US EPA 1668 for PCBs). Based on a combination of raw instrumental quantitative results, extraction solvent workup steps, original extraction volume, and starting specimen mass, the mass content of a trace chemical per mass of starting specimen material is calculated and is reported as the level of the trace chemical in the packaging film tested. Given the generally low levels of trace chemicals present, common units used in reported levels of trace chemicals are μg/kg and ng/kg (equivalent to the ppb and ppt levels, respectively).

One of skill in the art will recognize that trace chemicals are often present at very low "trace" levels, meaning that under some circumstances, environmental and/or laboratory background levels of these same trace chemicals may give rise to false positive detects or falsely elevated results if appropriate precautions are not taken. While one of skill in the art recognizes that a suite of measures may be needed to minimize such false results, one obligatory measure is to include a true "blank" beginning with solvent carried in parallel through the extraction step, extraction solvent workup, and instrumental analysis.

Using the suitable overall methodology outlined above, one of skill in the art may quantify a range of trace chemicals potentially present in a nonwoven or resin sample of interest. For example, the level of iso-Nonylphenol (CAS RN 11066-49-2) may be determined and reported as mass of trace chemical per mass of nonwoven or resin in units of μg/kg. Similarly, the level of iso-Nonylphenolhexaethoxylate (CAS RN 9016-45-9) may be determined and reported in units of μg/kg, the level of Di-2-ethylhexyl phthalate (CAS RN 117-81-7) may be determined and reported in units of μg/kg, the level of Octachlorodibenzodioxin (CAS RN 3268-87-9), a dioxin, may be determined and reported in units of ng/kg, the level of Naphtalene (CAS RN 91-20-3) may be determined and reported in units of μg/kg.

MFR (Melt Flow Rate) Test method is ASTM D1238. For clarity, this measurement is done at 230°C using a weight of 2.16kg and is the mass of material that comes out for 10 minute run time. Apparent Shear Viscosity Test method is ASTIM D3835 tested using a capillary rheometer at 230°C and a die with length to diameter ration of 30:1.

Tensile Strength Test Method

The CD tensile strength of a specimen is measured according to WSP 110.4-09 with conditions below.

Preload: 0.1N
Test Speed: 100 mm/min
Sample Width: 50 mm
Sample length: sufficiently longer than gauge length
Gauge Length: 100 mm

MD tensile strength of a specimen is measured according to WSP 110.4-09 with conditions below.

Preload: 0.1N
Test Speed: 100 mm/min
Sample Width: 50 mm
Sample length: sufficiently longer than gauge length
Gauge Length: 100 mm

Odor Analysis Test Method:

Odor sensory analysis is performed by placing about 3 g of each nonwoven sample to be tested in a 100mL glass vial with a sealing lid and equilibrating the sample within the sealed vial at room temperature for at least 24 hours. After equilibration, each vial is opened and the headspace is sniffed (bunny sniff) by a trained grader or a trained sensory panel of 7-10 graders (panel output would be a mean reported value) to determine odor intensity and descriptor profile. Odor intensity is graded according to the following scale:

5 = Very Strong

4 = Strong

3 = Moderate

2 = Weak to Moderate

1 = Weak

0 = No odor

Color and Opacity Measurement:

The color and opacity/translucency of a polymer and / or nonwoven are important parameters that determine whether or not a polymer can achieve the desired visual aesthetics of a nonwoven manufactured from the polymer as well as opacity/translucency of a nonwoven determines the desired visual aesthetics. Reclaimed polypropylene, especially post-consumer derived reclaimed polypropylene, is typically dark in color and opaque due to residual pigments, fillers, and other contamination. Thus, color and opacity measurements are important parameters in determining the effectiveness of a method for purifying polymers.

Prior to color measurement, samples of either polymeric powders or pellets were compression molded into 30 mm wide x 30 mm long x 1 mm thick square test specimens (with rounded corners). Powder samples were first densified at room temperature (ca. 20-23°C) by cold pressing the powder into a sheet using clean, un-used aluminum foil as a contact-release layer between stainless steel platens. Approximately 0.85 g of either cold-pressed powder or pellets was then pressed into test specimens on a Carver Press Model C (Carver, Inc., Wabash, IN 46992-0554 USA) pre-heated to 200°C using aluminum platens, unused aluminum foil release layers, and a stainless steel shim with a cavity corresponding to aforementioned dimensions of the square test specimens. Samples were heated for 5 minutes prior to applying pressure. After 5 minutes, the press was then compressed with at least 2 tons (1.81 metric tons) of hydraulic pressure for at least 5 seconds and then released. The molding stack was then removed and placed between two thick flat metal heat sinks for cooling. The aluminum foil contact release layers were then peeled from the sample and discarded. The flash around the sample on at least one side was peeled to the mold edge and then the sample was pushed through the form. Each test specimen was visually evaluated for voids/bubble defects and only samples with no defects in the color measurement area (0.7" (17.78 mm) diameter minimum) were used for color measurement.

For nonwovens, 10.16cm by 10.16cm portions of sample nonwoven ply were cut for analysis from a sample. The sample should be free from creases, wrinkles, tears, and other obvious defects for testing.

The color of each sample was characterized using the International Commission on Illumination (CIE) *L\*, a\*, b\** three dimensional color space. The dimension *L\** is a measure of the lightness of a sample, with *L\**=0 corresponding to the darkest black sample and *L\**=100 corresponding to the brightest white sample. The dimension *a\** is a measure of the red or green color of a sample with positive values of *a\** corresponding with a red color and negative values of *a\** corresponding with a green color. The dimension b\* is a measure of the blue or yellow color of a sample with positive values of *b\** corresponding with a yellow color and negative values of *b\** corresponding with a blue color. The *L\*a\*b\** values of each 30 mm wide x 30 mm long x 1 mm thick square test specimen sample were measured on a HunterLab model LabScan XE spectrophotometer (Hunter Associates Laboratory, Inc., Reston, VA 20190-5280, USA). The spectrophotometer was configured with D65 as the standard illuminant, an observer angle of 10°, an area diameter view of 1.75" (44.45 mm), and a port diameter of 0.7" (17.78 mm).

The opacity of each sample, which is a measure of how much light passes through the sample (i.e. a measure of the sample's translucency), was determined using the aforementioned HunterLab spectrophotometer using the contrast ratio opacity mode. Two measurements were made to determine the opacity of each sample. One to measure the brightness value of the sample backed with a white backing, $Y_{WhiteBacking}$, and one to measure the brightness value of the sample backed with a black backing, $Y_{BlackBacking}$. The opacity was then calculated from the brightness values using the following Equation 1:

Equation 1

$$\%Opacity = \frac{Y_{Black\ Backing}}{Y_{White\ Backing}} * 100$$

Basis Weight Test

Basis weight of the nonwoven webs described herein may be determined by several available techniques, but a simple representative technique involves taking an absorbent article or other consumer product, removing any elastic which may be present and stretching the absorbent article or other consumer product to its full length. A punch die having an area of 45.6 cm$^2$ is then used to cut a piece of the nonwoven web (e.g., topsheet, outer cover) from the approximate center of the absorbent article or other consumer product in a location which avoids to the greatest extent possible any adhesive which may be used to fasten the nonwoven web to any other layers which may be present and removing the nonwoven web from other layers (using cryogenic spray, such as Cyto-Freeze, Control Company, Houston, Texas, if needed). The sample is then weighed and dividing by the area of the punch die yields the basis weight of the nonwoven web. Results are reported as a mean of 5 samples to the nearest 0.1 gram per square meter (gsm).

Contemplated examples

I. A nonwoven having a total carbon content (TCC);

wherein from 10% to 100% of the TCC of the nonwoven are derived from waste carbon; and wherein the nonwoven meets one or more of the criteria a) to e) by comprising less than

a) 5000 μg/kg, preferably less than 100 μg/kg of iso-Nonylphenolhexaethoxylate;
b) 500 μg/kg, preferably less than 100 μg/kg of iso-Nonylphenol;
c) 4.0 ng/kg, preferably less than 0.4 ng/kg of Octachlorodibenzodioxin;
d) 500 μg/kg, preferably less than 100 μg/kg of Di-2-ethylhexyl phthalate; and/ or
e) 25 μg/kg, preferably less than 5 μg/kg of Napthalene;

according to the Trace Chemicals Test Method described herein.

II. The nonwoven of example I, wherein the nonwoven meets two or more, preferably three or more of the criteria a) to e).
III. The nonwoven of any one of examples I-II, wherein the nonwoven meets all of the criteria a) to e).
IV. The nonwoven of any one of examples I-III, wherein from 80% to 100%, preferably from 90% to 100%, more preferably from 95% to 100%, even more preferably from 97% to 100% or even from 99% to 100% of the TCC of the nonwoven are derived from waste carbon.
V. The nonwoven of any one of examples I-IV, wherein the TCC of the nonwoven derived from waste carbon is derived from recycled carbon.
VI. The nonwoven of any one of examples I-V, wherein the nonwoven is at least partially made from a resin obtained via solvent recycling of waste carbon.
VII. The nonwoven of any one examples I-VI, wherein the nonwoven is at least partially made from a resin meeting one or more of the criteria a) to e) by comprising less than

a) 5000 μg/kg, preferably less than 100 μg/kg of iso-Nonylphenolhexaethoxylate;
b) 500 μg/kg, preferably less than 100 μg/kg of iso-Nonylphenol;
c) 4.0 ng/kg, preferably less than 0.4 ng/kg of Octachlorodibenzodioxin;
d) 500 μg/kg, preferably less than 100 μg/kg of Di-2-ethylhexyl phthalate; and/ or
e) 25 μg/kg, preferably less than 5 μg/kg of Napthalene;

according to the Trace Chemicals Test Method described herein.
VIII. The nonwoven of any one of examples I-VII, wherein the nonwoven is made from a resin treated with a vis-breaking agent.
IX. The nonwoven of example VIII, wherein the vis-breaking agent is an oxidizing vis-breaking agent.
X. The nonwoven of examples VIII or IX, wherein the vis-breaking agent comprises a peroxide.
XI. The nonwoven of any one of examples I-II, wherein the nonwoven is at least partially made from a resin having a melt flow rate (MFR) from 20 g/10 min to 40 g/10 min, preferably from 25 g/10 min to 35 g/10min according to the Melt Flow Index Test Method described herein.
XII. The nonwoven of any one of examples I-XI, wherein the nonwoven is at least partially made from a resin having an Apparent Shear Viscosity from 15 Pa s to 150 Pa s, preferably from 20 Pa s to 100 Pa s according to the Apparent Shear Viscosity Test Method described herein.
XIII. The nonwoven of any one of examples I-XII, wherein the nonwoven has a polyolefin content of at least 90% by weight of the nonwoven.
XIV. The nonwoven of example XIII, wherein the polyolefin comprises one or more of low density polyethylene, high density polyethylene, medium density polyethylene, linear low density polyethylene, a propylene homopolymer, an ethylene copolymer, or a propylene copolymer or one or more blends thereof.

XV. The nonwoven of any one of examples I-XIV, wherein the nonwoven exhibits a machine direction (MD) to cross direction (CD) strength ratio of from 1.0 to 5.0, preferably from 1.2 to 3.5, more preferably from 1.5 to 2.5, even more preferably from 1.8 to 2.2 according to the Tensile Strength Test method described herein.

XVI. An absorbent article comprising a nonwoven of any one of examples I-IXV

XVII. Use of waste carbon to make a nonwoven having a total carbon content (TCC);

wherein from 10% to 100% of the TCC of the nonwoven are derived from the used waste carbon;
and wherein the nonwoven meets one or more of the criteria a) to e) by comprising less than

a) 5000 $\mu$g/kg, preferably less than 100 $\mu$g/kg of iso-Nonylphenolhexaethoxylate;
b) 500 $\mu$g/kg, preferably less than 100 $\mu$g/kg of iso-Nonylphenol;
c) 4.0 ng/kg, preferably less than 0.4 ng/kg of Octachlorodibenzodioxin;
d) 500 $\mu$g/kg, preferably less than 100 $\mu$g/kg of Di-2-ethylhexyl phthalate; and/ or
e) 25 $\mu$g/kg, preferably less than 5 $\mu$g/kg of Napthalene;

according to the Trace Chemicals Test Method described herein.

XVIII. The use of example XVII, wherein the nonwoven meets two or more, preferably three or more of the criteria a) to e).

XIX. The use of any one of examples XVII- XVIII, wherein the nonwoven meets all of the criteria a) to e).

XX. The use of any one of examples XVII- XIX, wherein from 80% to 100%, preferably from 90% to 100%, more preferably from 95% to 100%, even more preferably from 97% to 100% or even from 99% to 100% of the TCC of the nonwoven are derived from waste carbon.

XXI. The use of any one of examples XVII- XX, wherein the waste carbon is derived from recycled carbon.

XXII. The use of any one of examples XVII- XXI, wherein solvent recycled waste carbon is used to obtain a resin to make the nonwoven.

XXIII. The use of any one of examples XVII- XXII, wherein the waste carbon is used to obtain a resin meeting one or more of the criteria a) to e) by comprising less than

a) 5000 $\mu$g/kg, preferably less than 100 $\mu$g/kg of iso-Nonylphenolhexaethoxylate;
b) 500 $\mu$g/kg, preferably less than 100 $\mu$g/kg of iso-Nonylphenol;
c) 4.0 ng/kg, preferably less than 0.4 ng/kg of Octachlorodibenzodioxin;
d) 500 $\mu$g/kg, preferably less than 100 $\mu$g/kg of Di-2-ethylhexyl phthalate; and/ or
e) 25 $\mu$g/kg, preferably less than 5 $\mu$g/kg of Napthalene;

according to the Trace Chemicals Test Method described herein.

XXIV. The use of any one of claims examples XVII- XXIII, wherein the waste carbon is used to obtain a resin which is treated with a vis-breaking agent prior to making the nonwoven.

XXV. The use of example XXIV, wherein the vis-breaking agent is an oxidizing vis-breaking agent.

XXVI. The use of examples XXIV or XXV, wherein the vis-breaking agent comprises a peroxide.

XXVII. The use of any one of examples XVII- XXVI, wherein after vis-breaking the resin meets one or more of the criteria a) to e) by comprising less than

a) 5000 $\mu$g/kg, preferably less than 100 $\mu$g/kg of iso-Nonylphenolhexaethoxylate;
b) 500 $\mu$g/kg, preferably less than 100 $\mu$g/kg of iso-Nonylphenol;
c) 4.0 ng/kg, preferably less than 0.4 ng/kg of Octachlorodibenzodioxin;
d) 500 $\mu$g/kg, preferably less than 100 $\mu$g/kg of Di-2-ethylhexyl phthalate; and/ or
e) 25 $\mu$g/kg, preferably less than 5 $\mu$g/kg of Napthalene;

according to the Trace Chemicals Test Method described herein.

XXVIII. The use of any one of examples XVII- XXVII, wherein the waste carbon is used to obtain a resin having a melt flow rate (MFR) from 20 g/10 min to 40 g/10 min, preferably from 25 g/10 min to 35 g/10min according to the Melt Flow Index Test Method described herein to make the nonwoven.

XXIX. The use of any one of examples XVII- XXVIII, wherein the waste carbon is used to obtain a resin having an Apparent Shear Viscosity from 15 Pa s to 150 Pa s, preferably from 20 Pa s to 100 Pa s according to the Apparent Shear Viscosity Test Method described herein to make the nonwoven.

XXX. The use of any one of examples XVII- XXIX, wherein the nonwoven has a polyolefin content of at least 90% by weight of the nonwoven.

XXXI. The use of example XXX, wherein the polyolefin comprises one or more of low density polyethylene, high density polyethylene, medium density polyethylene, linear low density polyethylene, a propylene homopolymer, an ethylene copolymer, or a propylene copolymer or one or more blends thereof.

XXXII. The use of any one of examples XVII- XXXI, wherein the nonwoven exhibits a machine direction (MD) to cross direction (CD) strength ratio of from 1.0 to 5.0, preferably from 1.2 to 3.5, more preferably from 1.5 to 2.5, even more preferably from 1.8 to 2.2 according to the Tensile Strength Test method described herein.

XXXIII. A method for making a nonwoven, wherein the nonwoven has a total carbon content (TCC);

wherein waste carbon is provided;
wherein in the made nonwoven from 10% to 100% of the TCC derive from the provided waste carbon;
and wherein the made nonwoven meets one or more of the criteria a) to e) by comprising less than

a) 5000 $\mu$g/kg, preferably less than 100 $\mu$g/kg of iso-Nonylphenolhexaethoxylate;
b) 500 $\mu$g/kg, preferably less than 100 $\mu$g/kg of iso-Nonylphenol;
c) 4.0 ng/kg, preferably less than 0.4 ng/kg of Octachlorodibenzodioxin;
d) 500 $\mu$g/kg, preferably less than 100 $\mu$g/kg of Di-2-ethylhexyl phthalate; and/ or
e) 25 $\mu$g/kg, preferably less than 5 $\mu$g/kg of Napthalene;

according to the Trace Chemicals Test Method described herein.

XXXIV. The method of example XXXIII, wherein the nonwoven meets two or more, preferably three or more of the criteria a) to e).

XXXV. The method of any one of examples XXXIII- XXXIV, wherein the nonwoven meets all of the criteria a) to e).

XXXVI. The method of any one of examples XXXIII- XXXV, wherein from 80% to 100%, preferably from 90% to 100%, more preferably from 95% to 100%, even more preferably from 97% to 100% or even from 99% to 100% of the TCC of the nonwoven are derived from waste carbon.

XXXVII. The method of any one of examples XXXIII- XXXVI, wherein the waste carbon is derived from recycled carbon.

XXXVIII. The method of any one of examples XXXIII- XXXVII, comprising the step of solvent recycling the waste carbon to obtain a resin to make the nonwoven.

XXXIX. The method of any one of examples XXXIII- XXXVIII, comprising the step of obtaining a resin from the waste carbon meeting one or more of the criteria a) to e) by comprising less than

a) 5000 $\mu$g/kg, preferably less than 100 $\mu$g/kg of iso-Nonylphenolhexaethoxylate;
b) 500 $\mu$g/kg, preferably less than 100 $\mu$g/kg of iso-Nonylphenol;
c) 4.0 ng/kg, preferably less than 0.4 ng/kg of Octachlorodibenzodioxin;
d) 500 $\mu$g/kg, preferably less than 100 $\mu$g/kg of Di-2-ethylhexyl phthalate; and/ or
e) 25 $\mu$g/kg, preferably less than 5 $\mu$g/kg of Napthalene;

according to the Trace Chemicals Test Method described herein.

XL. The method of any one of examples XXXIII-XXXIX, comprising the step of treating a resin obtained from the waste carbon with a vis-breaking agent.

XLI. The method of example XL, wherein the vis-breaking agent is an oxidizing vis-breaking agent.

XLII. The method of examples XL or XLI, wherein the vis-breaking agent comprises a peroxide.

XLIII. The method of any one of claims examples XI,-XI,II, wherein prior to the step of vis-breaking the resin meets one or more of the criteria a) to e) by comprising less than

a) 5000 $\mu$g/kg, preferably less than 100 $\mu$g/kg of iso-Nonylphenolhexaethoxylate;
b) 500 $\mu$g/kg, preferably less than 100 $\mu$g/kg of iso-Nonylphenol;
c) 4.0 ng/kg, preferably less than 0.4 ng/kg of Octachlorodibenzodioxin;
d) 500 $\mu$g/kg, preferably less than 100 $\mu$g/kg of Di-2-ethylhexyl phthalate; and/ or
e) 25 $\mu$g/kg, preferably less than 5 $\mu$g/kg of Napthalene;

according to the Trace Chemicals Test Method described herein.

XLIV. The method of any one of examples XI,-XI,III, wherein after to the step of vis-breaking the resin meets one or more of the criteria a) to e) by comprising less than

a) 5000 μg/kg, preferably less than 100 μg/kg of iso-Nonylphenolhexaethoxylate;
b) 500 μg/kg, preferably less than 100 μg/kg of iso-Nonylphenol;
c) 4.0 ng/kg, preferably less than 0.4 ng/kg of Octachlorodibenzodioxin;
d) 500 μg/kg, preferably less than 100 μg/kg of Di-2-ethylhexyl phthalate; and/ or
e) 25 μg/kg, preferably less than 5 μg/kg of Napthalene;

according to the Trace Chemicals Test Method described herein.

XLV. The method of any one of examples XXXIII-XLIV, comprising the step of obtaining a resin having a melt flow rate (MFR) from 20 g/10 min to 40 g/10 min, preferably from 25 g/10 min to 35 g/10min according to the Melt Flow Index Test Method described herein from the waste carbon .

XLVI. The method of any one of examples XXXIII-XLV, comprising the step of obtaining a resin having an Apparent Shear Viscosity from 15 Pa s to 150 Pa s, preferably from 20 Pa s to 100 Pa s according to the Apparent Shear Viscosity Test Method described herein from the waste carbon.

XLVII. The method of any one of examples XXXIII-XLVI, wherein the nonwoven has a polyolefin content of at least 90% by weight of the nonwoven.

XLVIII. The method of example XI,VII, wherein the polyolefin comprises one or more of low density polyethylene, medium density polyethylene high density polyethylene, linear low density polyethylene, a propylene homopolymer, an ethylene copolymer, or a propylene copolymer or one or more blends thereof.

XLIX. The method of any one of examples XXXIII-XLVIII, wherein the nonwoven exhibits a machine direction (MD) to cross direction (CD) strength ratio of from 1.0 to 5.0, preferably from 1.2 to 3.5, more preferably from 1.5 to 2.5, even more preferably from 1.8 to 2.2 according to the Tensile Strength Test method described herein.

XLX. The nonwoven of any one of examples I-XV, wherein the nonwoven exhibits an odor intensity score of less than 2, less than 1 or even 0 according to the Odor Analysis Test Method described herein.

XLXII.The use of any one of examples XVII- XXXII, wherein the nonwoven exhibits an odor intensity score of less than 2, less than 1 or even 0 according to the Odor Analysis Test Method described herein.

XLXII. The method of any one of examples XXXIII-XLIX, wherein the nonwoven exhibits an odor intensity score of less than 2, less than 1 or even 0 according to the Odor Analysis Test Method described herein.

XLXIII. The nonwoven of any one of examples I-XV or XLXI, wherein the nonwoven exhibits an opacity of from 5% to 70%, preferably from 10% to 60%, more preferably from 15 to 55% according to the Color and Opacity Measurement.

XIXIV. The nonwoven of any one of examples I-XV, XLXI or XLXIII, wherein the nonwoven exhibits a L* value of at least 85, preferably at least 88, more preferably at least 90, even more preferably at least 92, most preferably at least 94 or even at least 96 according to the Color and Opacity Measurement.

XLXV. The nonwoven of any one of examples I-XV, XLXI or XI,XIII-XI,XIV, wherein the nonwoven exhibits an a* value of from -20 to 20, preferably from -15 to 15, more preferably from -10 to 10, even more preferably from -5 to 5, most preferably from -3 to 3 or even around 0 according to the Color and Opacity Measurement.

XLXVI. The nonwoven of any one of examples I-XV, XLXI or XLXIII-XLXV, wherein the nonwoven exhibits a b* value of from -20 to 30, preferably from -15 to 20, more preferably from -10 to 10, even more preferably from -5 to 5, most preferably from -3 to 3 or even around 0 according to the Color and Opacity Measurement.

XLXVII. The use of any one of examples XVII- XXXII or XLXXII, wherein the nonwoven exhibits an opacity of from 5% to 70%, preferably from 10% to 60%, more preferably from 15 to 55% according to the Color and Opacity Measurement.

XLXVIII. The use of any one of examples XVII- XXXII, XLXXII or XLXVIII, wherein the nonwoven exhibits a L* value of at least 85, preferably at least 88, more preferably at least 90, even more preferably at least 92, most preferably at least 94 or even at least 96 according to the Color and Opacity Measurement.

XLXIX. The use of any one of examples XVII- XXXII, XLXXII or XLXVII- XLXVIII, wherein the nonwoven exhibits an a* value of from -20 to 20, preferably from -15 to 15, more preferably from -10 to 10, even more preferably from -5 to 5, most preferably from -3 to 3 or even around 0 according to the Color and Opacity Measurement.

XLXX. The use of any one of examples XVII- XXXII, XLXXII or XLXVII- XLXIX, wherein the nonwoven exhibits a b* value of from -20 to 30, preferably from -15 to 20, more preferably from -10 to 10, even more preferably from -5 to 5, most preferably from -3 to 3 or even around 0 according to the Color and Opacity Measurement.

XLXXI. The method of any one of examples XXXIII-XLIX or XI,XXIII, wherein the nonwoven exhibits an opacity of from 5% to 70%, preferably from 10% to 60%, more preferably from 15 to 55% according to the Color and Opacity Measurement.

XLXXII. The method of any one of examples XXXIII-XLIX, XLXXIII or XLXXI, wherein the nonwoven exhibits a L* value of at least 85, preferably at least 88, more preferably at least 90, even more preferably at least 92, most preferably at least 94 or even at least 96 according to the Color and Opacity Measurement.

XLXXIII. The method of any one of examples XXXIII-XLIX, XLXXIII or XLXXI- XLXXII, wherein the nonwoven exhibits an a* value of from -20 to 20, preferably from -15 to 15, more preferably from -10 to 10, even more preferably from -5 to 5, most preferably from -3 to 3 or even around 0 according to the Color and Opacity Measurement.

XLXXIV. The method of any one of examples XXXIII-XLIX, XLXXIII or XLXXI- XLXXIII, wherein the nonwoven exhibits an b* value of from -20 to 30, preferably from -15 to 20, more preferably from -10 to 10, even more preferably from -5 to 5, most preferably from -3 to 3 or even around 0 according to the Color and Opacity Measurement.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. A nonwoven having a total carbon content (TCC);

   wherein from 10% to 100% of the TCC of the nonwoven are derived from waste carbon;
   and wherein the nonwoven meets one or more of the criteria a) to e) by comprising less than

   a) 5000 $\mu$g/kg, preferably less than 100 $\mu$g/kg of iso-Nonylphenolhexaethoxylate;
   b) 500 $\mu$g/kg, preferably less than 100 $\mu$g/kg of iso-Nonylphenol;
   c) 4.0 ng/kg, preferably less than 0.4 ng/kg of Octachlorodibenzodioxin;
   d) 500 $\mu$g/kg, preferably less than 100 $\mu$g/kg of Di-2-ethylhexyl phthalate; and/ or
   e) 25 $\mu$g/kg, preferably less than 5 $\mu$g/kg of Napthalene;

   according to the Trace Chemicals Test Method described herein.

2. The nonwoven of claim 1, wherein the nonwoven meets two or more, preferably three or more, more preferably all of the criteria a) to e)).

3. The nonwoven of any one of the preceding claims, wherein from 80% to 100%, preferably from 90% to 100%, more preferably from 95% to 100%, even more preferably from 97% to 100% or even from 99% to 100% of the TCC of the nonwoven are derived from waste carbon.

4. The nonwoven of any one of the preceding claims, wherein the TCC of the nonwoven derived from waste carbon is derived from recycled carbon, preferably post-consumer recycled carbon.

5. The nonwoven of any one of the preceding claims, wherein the nonwoven is at least partially made from a resin obtained via solvent recycling of waste carbon.

6. The nonwoven of any one of the preceding claims, wherein the nonwoven is made from a resin meeting one or more of the criteria a) to e) by comprising less than

   a) 5000 $\mu$g/kg, preferably less than 100 $\mu$g/kg of iso-Nonylphenolhexaethoxylate;
   b) 500 $\mu$g/kg, preferably less than 100 $\mu$g/kg of iso-Nonylphenol;
   c) 4.0 ng/kg, preferably less than 0.4 ng/kg of Octachlorodibenzodioxin;
   d) 500 $\mu$g/kg, preferably less than 100 $\mu$g/kg of Di-2-ethylhexyl phthalate; and/ or
   e) 25 $\mu$g/kg, preferably less than 5 $\mu$g/kg of Napthalene;

   according to the Trace Chemicals Test Method described herein.

7. The nonwoven of any one of the preceding claims, wherein the nonwoven is at least partially made from a resin treated with a vis-breaking agent, preferably an oxidizing vis-breaking agent, more preferably a vis-breaking agent comprising a peroxide.

8. The nonwoven of any one of the preceding claims, wherein the nonwoven is at least partially made from a resin having a melt flow rate (MFR) from 20 g/10 min to 60 g/10 min, preferably from 25 g/10 min to 50 g/10min according to the Melt Flow Index Test Method described herein.

9. The nonwoven of any one of the preceding claims, wherein the nonwoven is at least partially made from a resin having an Apparent Shear Viscosity from 15 Pa s to 150 Pa s, preferably from 20 Pa s to 100 Pa s according to the Apparent Shear Viscosity Test Method described herein.

10. The nonwoven of any one of the preceding claims, wherein the nonwoven has a polyolefin content of at least 90% by weight of the nonwoven, preferably wherein the polyolefin comprises one or more of low density polyethylene, high density polyethylene, medium density polyethylene, linear low density polyethylene, a propylene homopolymer, an ethylene copolymer, or a propylene copolymer or one or more blends thereof.

11. The nonwoven of any one of the preceding claims, wherein the nonwoven exhibits a machine direction (MD) to cross direction (CD) strength ratio of from 1.0 to 5.0, preferably from 1.2 to 3.5, more preferably from 1.5 to 2.5, even more preferably from 1.8 to 2.2 according to the Tensile Strength Test method described herein.

12. The nonwoven of any one of the preceding claims, wherein the nonwoven is a spunbonded nonwoven.

13. An absorbent article comprising a nonwoven of any one of the preceding claims.

14. Use of waste carbon to make a nonwoven having a total carbon content (TCC);

  wherein from 10% to 100% of the TCC of the nonwoven are derived from the used waste carbon;
  and wherein the nonwoven meets one or more of the criteria a) to e) by comprising less than

    a) 5000 $\mu$g/kg, preferably less than 100 $\mu$g/kg of iso-Nonylphenolhexaethoxylate;
    b) 500 $\mu$g/kg, preferably less than 100 $\mu$g/kg of iso-Nonylphenol;
    c) 4.0 ng/kg, preferably less than 0.4 ng/kg of Octachlorodibenzodioxin;
    d) 500 $\mu$g/kg, preferably less than 100 $\mu$g/kg of Di-2-ethylhexyl phthalate; and/ or
    e) 25 $\mu$g/kg, preferably less than 5 $\mu$g/kg of Napthalene;

  according to the Trace Chemicals Test Method described herein.

15. A method for making a nonwoven, wherein the nonwoven has a total carbon content (TCC);

  wherein waste carbon is provided;
  wherein in the made nonwoven from 10% to 100% of the TCC derive from the provided waste carbon;
  and wherein the made nonwoven meets one or more of the criteria a) to e) by comprising less than

    a) 5000 $\mu$g/kg, preferably less than 100 $\mu$g/kg of iso-Nonylphenolhexaethoxylate;
    b) 500 $\mu$g/kg, preferably less than 100 $\mu$g/kg of iso-Nonylphenol;
    c) 4.0 ng/kg, preferably less than 0.4 ng/kg of Octachlorodibenzodioxin;
    d) 500 $\mu$g/kg, preferably less than 100 $\mu$g/kg of Di-2-ethylhexyl phthalate; and/ or
    e) 25 $\mu$g/kg, preferably less than 5 $\mu$g/kg of Napthalene;

  according to the Trace Chemicals Test Method described herein.

16. The method of any one claim 15 comprising the step of solvent recycling the waste carbon to obtain a resin to make the nonwoven.

17. The method of any one of claims 15 or 16, comprising the step of treating a resin obtained from the waste carbon with a vis-breaking agent, preferably wherein the vis-breaking agent is an oxidizing vis-breaking agent, more preferably wherein the vis-breaking agent comprises a peroxide.

18. The method of claim 16, wherein prior to the step of vis-breaking the resin meets one or more of the criteria a) to e) by comprising less than

    a) 5000 $\mu$g/kg, preferably less than 100 $\mu$g/kg of iso-Nonylphenolhexaethoxylate;
    b) 500 $\mu$g/kg, preferably less than 100 $\mu$g/kg of iso-Nonylphenol;
    c) 4.0 ng/kg, preferably less than 0.4 ng/kg of Octachlorodibenzodioxin;
    d) 500 $\mu$g/kg, preferably less than 100 $\mu$g/kg of Di-2-ethylhexyl phthalate; and/ or

e) 25 μg/kg, preferably less than 5 μg/kg of Napthalene;

according to the Trace Chemicals Test Method described herein.

**19.** The method of claim 16, wherein after to the step of vis-breaking the resin meets one or more of the criteria a) to e) by comprising less than

a) 5000 μg/kg, preferably less than 100 μg/kg of iso-Nonylphenolhexaethoxylate;
b) 500 μg/kg, preferably less than 100 μg/kg of iso-Nonylphenol;
c) 4.0 ng/kg, preferably less than 0.4 ng/kg of Octachlorodibenzodioxin;
d) 500 μg/kg, preferably less than 100 μg/kg of Di-2-ethylhexyl phthalate; and/ or
e) 25 μg/kg, preferably less than 5 μg/kg of Napthalene;

according to the Trace Chemicals Test Method described herein.

Fig. 1

Fig. 2

Fig. 3

Fig. 4

Fig. 5

Fig. 6

Fig. 7

Fig. 8

Fig. 9

Fig. 10

Fig. 11

Fig. 12

EP 4 745 282 A1

Fig. 13

## EUROPEAN SEARCH REPORT

**Application Number**

EP 24 21 3146

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2024/211679 A1 (BERRY GLOBAL INC [US]) 10 October 2024 (2024-10-10) * claims 1, 14, 15 * | 1-6 | INV. D04H1/4291 D04H1/4274 D04H3/007 |
| Y | EP 3 339 362 A1 (PROCTER & GAMBLE [US]) 27 June 2018 (2018-06-27) * paragraphs [0052], [0055]; claims 1, 6, 11 * | 1-6 | D04H3/16 C08F8/00 C08F210/06 C08L23/12 |
| A | WO 2015/112844 A1 (FITESA SIMPSONVILLE INC [US]; FITESA GERMANY GMBH [DE]) 30 July 2015 (2015-07-30) * examples * | 1-6 | |
| A | WO 00/40789 A1 (KIMBERLY CLARK CO [US]) 13 July 2000 (2000-07-13) * examples * | 1-6 | |
| A | US 2023/167251 A1 (KOHLWEYER CHRISTIAN [DE] ET AL) 1 June 2023 (2023-06-01) * claims 1, 7, 12 * | 1-6 | TECHNICAL FIELDS SEARCHED (IPC) D04H C08L C08F |

The present search report has been drawn up for all claims

| Place of search | Date of completion of the search | Examiner |
|---|---|---|
| Munich | 20 June 2025 | Saunders, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 3146

This annex lists the patent family members relating to the patent documents cited in the above-mentioned European search report.
The members are as contained in the European Patent Office EDP file on
The European Patent Office is in no way liable for these particulars which are merely given for the purpose of information.

20-06-2025

| Patent document cited in search report | | | Publication date | Patent family member(s) | | Publication date |
|---|---|---|---|---|---|---|
| WO 2024211679 | A1 | | 10-10-2024 | US | 2024337051 A1 | 10-10-2024 |
| | | | | WO | 2024211679 A1 | 10-10-2024 |
| EP 3339362 | A1 | | 27-06-2018 | EP | 3339362 A1 | 27-06-2018 |
| | | | | ES | 2949849 T3 | 03-10-2023 |
| | | | | US | 2018171097 A1 | 21-06-2018 |
| | | | | US | 2020062922 A1 | 27-02-2020 |
| WO 2015112844 | A1 | | 30-07-2015 | BR | 112016017217 A2 | 08-08-2017 |
| | | | | CN | 106103824 A | 09-11-2016 |
| | | | | DK | 3097224 T3 | 02-01-2019 |
| | | | | EP | 3097224 A1 | 30-11-2016 |
| | | | | ES | 2700363 T3 | 15-02-2019 |
| | | | | JP | 6297719 B2 | 20-03-2018 |
| | | | | JP | 2017508082 A | 23-03-2017 |
| | | | | MX | 361065 B | 26-11-2018 |
| | | | | PE | 20160919 A1 | 03-09-2016 |
| | | | | PL | 3097224 T3 | 28-02-2019 |
| | | | | TR | 201815465 T4 | 21-11-2018 |
| | | | | US | 2016333509 A1 | 17-11-2016 |
| | | | | WO | 2015112844 A1 | 30-07-2015 |
| WO 0040789 | A1 | | 13-07-2000 | AU | 2721100 A | 24-07-2000 |
| | | | | US | 6583076 B1 | 24-06-2003 |
| | | | | WO | 0040789 A1 | 13-07-2000 |
| US 2023167251 | A1 | | 01-06-2023 | US | 2023167251 A1 | 01-06-2023 |
| | | | | US | 2023416481 A1 | 28-12-2023 |
| | | | | US | 2025179259 A1 | 05-06-2025 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10450436 B2
- US 10442912 B2
- US 20140005020
- US 9421137 B
- US 9072632 B
- US 7901393 B
- US 9498389 A
- US 6645190 B
- US 8747379 B
- US 8372052 B
- US 8361048 B
- US 6761711 B
- US 6817994 B
- US 8007485 B
- US 7862550 B
- US 6969377 B
- US 7497851 B
- US 6849067 B
- US 6893426 B
- US 6953452 B
- US 6840928 B
- US 8579876 B
- US 7682349 B
- US 7156833 B
- US 7201744 B
- US 20170191198 A
- US 5628097 A, Benson
- US 20160136014 A, Arora
- WO 2023022979 A
- US 5599335 A
- EP 1447066 A
- WO 9511652 A
- US 20080312622 A1
- WO 2012052172 A
- US 7838722 B
- US 9072634 B
- US 8206533 B
- US 9789011 B
- US 10485713 B
- US 8784722 B, Rocha
- US 20070219521 A1
- US 20070219521
- US 8703450 B
- US 9630901 B
- US 9822197 B
- US 20110139657
- US 20110139658
- US 20110152812
- US 10166312 B
- US 9169366 B
- US 11433158 B

**Non-patent literature cited in the description**

- **S.M. AL-SALEM** ; **P. LETTIERI** ; **J. BAEYENS**. Recycling and recovery routes of plastic solid waste (PSW): A review. *Waste Management*, October 2009, vol. 29 (10), ISSN 0956-053X, 2625-2643
- *CHEMICAL ABSTRACTS*, 11066-49-2
- *CHEMICAL ABSTRACTS*, 9016-45-9
- *CHEMICAL ABSTRACTS*, 117-81-7
- *CHEMICAL ABSTRACTS*, 3268-87-9
- *CHEMICAL ABSTRACTS*, 91-20-3